# EUROPEAN PATENT APPLICATION

(11) **EP 4 803 632 A1**
(43) Date of publication of application: **09.09.2026**
(21) Application number: 24885887.0
(22) Date of filing: 01.11.2024
(51) Int. Cl.: C12P 7/40, A23C 20/00, A23L 23/00, A23L 27/00, C12N 1/20, C12P 7/26

(54) **METHOD FOR IMPROVING FLAVOR**

(30) Priority: 02.11.2023 JP 2023188588
(71) Applicant: AJINOMOTO CO., INC., Chuo-ku Tokyo 104-8315 (JP)
(72) Inventor: HAYASHI, Kazuyuki, Kawasaki-shi, Kanagawa 210-8681 (JP); MAEDA, Miyoko, Kawasaki-shi, Kanagawa 210-8681 (JP); IWAMI, Kotaro, Kawasaki-shi, Kanagawa 210-8681 (JP); MIZUNO, Masaki, Kawasaki-shi, Kanagawa 210-8681 (JP)
(74) Representative: Strehl & Partner mbB
(86) International application number: PCT/JP2024/039150
(87) International publication number: WO 2025/095123

(57) **Abstract**

A technique for improving flavor of foods is provided. According to the present invention, *Bacillus* bacteria are used to produce components effective for improving flavor of foods, such as 2-methylbutyric acid, diacetyl, and acetoin.

## Description

### Technical Field

The present invention relates to a technique for improving flavor of a food. Specifically, the present invention relates to a food flavor improver and a method for improving flavor of a food.

### Background Art

Aliphatic carboxylic acids such as 2-methylbutyric acid, 3-methylbutyric acid, and isobutyric acid, diacetyl, and acetoin are known as components of cheese (Non-patent document 1).

A method for producing (S)-2-methylbutyric acid from L-isoleucine using *Bacillus* bacteria has been reported (Patent document 1). Methods for producing aliphatic carboxylic acids using yeast have also been reported (Patent documents 2 to 5, Non-patent document 2).

A method for producing diacetyl and acetoin using *Bacillus* bacteria has been reported (Non-patent document 3).

### Prior Art References

### Patent documents

Patent document 1: JP 2009-284861 A
Patent document 2: JP 2020-156343 A
Patent document 3: JP 2013-223485 A
Patent document 4: JP H06-504447 A
Patent document 5: JP S61-092582 A

### Non-patent documents

Non-patent document 1: T. K. Singh et al., Flavor of Cheddar Cheese: A Chemical and Sensory Perspective, COMPREHENSIVE REVIEWS IN FOOD SCIENCE AND FOOD SAFETY - Vol. 2, 2003
Non-patent document 2: Weerawat Runguphan and Jay D Keasling, Metabolic engineering of Saccharomyces cerevisiae for production of fatty acid-derived biofuels and chemicals, Metab Eng. 2014 Jan;21:103-13
Non-patent document 3: Kaloyan Petrov and Penka Petrova, Current Advances in Microbial Production of Acetoin and 2,3-Butanediol by Bacillus spp., Fermentation 2021, 7(4), 307

### Summary of the Invention

### Object to be achieved by the invention

An object of the present invention is to provide a technique for improving flavor of foods.

### Means for achieving the object

The inventors of the present invention conducted diligent investigation to achieve the above object, as a result, discovered that components effective for improving flavors of foods can be produced by using bacteria of the genus *Bacillus*, and thereby accomplished the present invention.

Specifically, the present invention can be embodied, for example, as follows.
[1] A method for producing a carboxylic acid, diacetyl, and acetoin, comprising
   the step of culturing a *Bacillus* bacterium having a carboxylic acid-producing ability, a diacetyl-producing ability, and an acetoin-producing ability in a culture medium containing a precursor of the carboxylic acid and a sugar to obtain a culture containing the carboxylic acid, diacetyl, and acetoin, wherein
   the carboxylic acid is selected from the group consisting of 2-methylbutyric acid, 3-methylbutyric acid, isobutyric acid, and a combination thereof,
   the precursor of 2-methylbutyric acid is isoleucine, the precursor of 3-methylbutyric acid is leucine, and the precursor of isobutyric acid is selected from the group consisting of isoleucine, leucine, valine, and a combination thereof.
[2] The method mentioned above (specifically, according to [1]), wherein 2-methylbutyric acid is (S)-2-methylbutyric acid and/or (R)-2-methylbutyric acid, and the precursor of (S)-2-methylbutyric acid and the precursor of (R)-2-methylbutyric acid are L-isoleucine and D-isoleucine, respectively.
[3] The method mentioned above (specifically, according to [1] or [2]), wherein the carboxylic acid, diacetyl, and acetoin are produced as a composition comprising the carboxylic acid, diacetyl, and acetoin.
[4] The method mentioned above (specifically, according to [3]), wherein the composition comprises the culture or a processed product thereof.
[5] The method mentioned above (specifically, according to [3] or [4]), wherein the composition comprises a dried product of the culture or a dried product of the supernatant of the culture.
[6] The method mentioned above (specifically, according to any of [3] to [5]), wherein content of the carboxylic acid in the composition is 10 ppm (w/w) or higher.
[7] The method mentioned above (specifically, according to any of [3] to [6]), wherein content of diacetyl in the composition is 0.5 ppm (w/w) or higher.
[8] The method mentioned above (specifically, according to any of [3] to [7]), wherein content of acetoin in the composition is 10 ppm (w/w) or higher.
[9] The method mentioned above (specifically, according to any of [1] to [8]), wherein the bacterium is *Bacillus subtilis*, *Bacillus amyloliquefaciens*, *Bacillus pumilus*, *Bacillus licheniformis*, *Bacillus megaterium*, *Bacillus brevis*, *Bacillus polymixa*, *Bacillus stearothermophilus*, or *Bacillus velezensis*.
[10] The method mentioned above (specifically, according to any of [1] to [9]), wherein the bacterium is *Bacillus subtilis* or *Bacillus amyloliquefaciens*.
[11] The method mentioned above (specifically, according to any of [1] to [10]), wherein isoleucine content in the medium is 0.1 to 5% (w/w).
[12] The method mentioned above (specifically, according to any of [1] to [11]), wherein leucine content in the medium is 0.1 to 5% (w/w).
[13] The method mentioned above (specifically, according to any of [1] to [12]), wherein valine content in the medium is 0.1 to 5% (w/w).
[14] The method mentioned above (specifically, according to any of [1] to [13]), wherein content of the sugar in the medium is 1 to 50% (w/w).
[15] The method mentioned above (specifically, according to any of [1] to [14]), wherein the sugar is glucose.
[16] The method mentioned above (specifically, according to any of [3] to [15]), wherein the composition is a composition for improving flavor of a food.
[17] The method mentioned above (specifically, according to [16]), wherein the improvement of flavor is imparting cheese flavor.
[18] The method mentioned above (specifically, according to any of [3] to [17]), wherein the composition is a seasoning.
[19] The method mentioned above (specifically, according to any of [1] to [18]), wherein at least 2-methylbutyric acid is produced.
[20] The method mentioned above (specifically, according to [19]), wherein 3-methylbutyric acid is further produced.
[21] The method mentioned above (specifically, according to [19] or [20]), wherein isobutyric acid is further produced.
[22] A composition produced by the method mentioned above (specifically, according to any of [3] to [21]).
[23] A composition for improving flavor of a food, comprising
   the following components (A), (B), and (C):
   (A) a carboxylic acid selected from the group consisting of 2-methylbutyric acid, 3-methylbutyric acid, isobutyric acid, and a combination thereof;
   (B) diacetyl; and
   (C) acetoin.
[24] The composition mentioned above (specifically, according to [23]), wherein the components (A), (B), and (C) have been produced by the method mentioned above (specifically, according to any of [1] to [21]).
[25] The composition mentioned above (specifically, according to [23] or [24]), which comprises at least 2-methylbutyric acid.
[26] The composition mentioned above (specifically, according to [25]), which further comprises 3-methylbutyric acid.
[27] The composition mentioned above (specifically, according to [25] or [26]), which further comprises isobutyric acid.
[28] The composition mentioned above (specifically, according to any of [23] to [27]), wherein the improvement of flavor is imparting cheese flavor.
[29] A method for improving flavor of a food, comprising:
   the step of adding the following components (A), (B), and (C) to a raw material of the food:
   (A) a carboxylic acid selected from the group consisting of 2-methylbutyric acid, 3-methylbutyric acid, isobutyric acid, and a combination thereof;
   (B) diacetyl; and
   (C) acetoin.
[30] The method mentioned above (specifically, according to [29]), wherein the components (A), (B), and (C) have been produced by the method mentioned above (specifically, according to any of [1] to [21]).
[31] The method mentioned above (specifically, according to [29] or [30]), which comprises the step of producing the components (A), (B), and (C) by the method mentioned above (specifically, according to any of [1] to [21]) prior to the step mentioned above.
[32] The method mentioned above (specifically, according to any of [29] to [31]), wherein the component (A) is added so that the concentration thereof at the time of eating is 0.001 to 5,000 ppm (w/w).
[33] The method mentioned above (specifically, according to any of [29] to [32]), wherein the component (B) is added so that the concentration thereof at the time of eating is 0.0001 to 100 ppm (w/w).
[34] The method mentioned above (specifically, according to any of [29] to [33]), wherein the component (C) is added so that the concentration thereof at the time of eating is 0.001 to 5,000 ppm (w/w).
[35] The method mentioned above (specifically, according to any of [29] to [34]), wherein at least 2-methylbutyric acid is added.
[36] The method mentioned above (specifically, according to [35]), wherein 3-methylbutyric acid is further added.
[37] The method mentioned above (specifically, according to [35] or [36]), wherein isobutyric acid is further added.
[38] The method mentioned above (specifically, according to any of [29] to [37]), wherein the improvement of flavor is imparting cheese flavor.

### Effect of the Invention

According to the present invention, flavor of foods can be improved.

### Embodiments for Carrying out the Invention

Hereafter, the present invention will be explained in detail.

### <1> Active ingredient

### <1-1> Active Ingredient

In the present invention, the following components (A), (B), and (C) are used as active ingredients:
(A) a carboxylic acid selected from the group consisting of 2-methylbutyric acid, 3-methylbutyric acid, isobutyric acid, and a combination thereof;
(B) diacetyl; and
(C) acetoin.

The components (A), (B), and (C) are also collectively referred to as "active ingredient". The component (A) is also referred to as "carboxylic acid".

By utilizing the active ingredient, flavor of foods can be improved, i.e., an effect of improving flavor of foods can be obtained. This effect is also referred to as "flavor-improving effect". In other words, the active ingredient has a function to improve flavor of foods. This function is also referred to as the "flavor-improving function". The improvement of flavor of foods is also simply referred to as "improvement of flavor". An example of the improvement of flavor is impartation of cheese flavor to foods. That is, an example of the flavor-improving effect is an effect of imparting cheese flavor to foods. This effect is also referred to as "cheese flavor-imparting effect". Further, an example of the flavor-improving function is a function of imparting cheese flavor to foods. This function is also referred to as "cheese flavor-imparting function". Impartation of cheese flavor to foods is also simply referred to as "impartation of cheese flavor". The "impartation of cheese flavor" includes impartation of cheese flavor to foods lacking cheese flavor (e.g., foods not containing cheese) and enhancing cheese flavor of foods having cheese flavor (e.g., foods containing cheese). Examples of the cheese flavor include the characteristic rich aroma and characteristic richness of cheese. Specifically, by utilizing the active ingredient, flavor of foods can be improved compared with when the active ingredient is not utilized (e.g., cheese flavor can be imparted to foods). Therefore, the flavor-improving effect (e.g., cheese flavor-imparting effect) can be determined by measuring and comparing flavor of foods (e.g., cheese flavor of foods) not utilizing the active ingredient and flavor of the same types of foods utilizing the active ingredient. That is, when flavor of a food utilizing the active ingredient is more preferable compared with the food not utilizing the active ingredient, it can be determined that the flavor-improving effect is obtained. Specifically, for example, when cheese flavor of a food utilizing the active ingredient is stronger than that of the food not utilizing the active ingredient, it can be determined that the cheese flavor-imparting effect is obtained. Examples of foods not utilizing the active ingredient include foods utilizing none of the components (A) to (C), and foods not utilizing a part of the components (A) to (C). That is, by using at least the components (A) to (C) in combination, flavor of a food can be improved compared with the food utilizing none of the components (A) to (C) (e.g., cheese flavor can be imparted to the food). Further, in one embodiment, by using the components (A) to (C) in combination, flavor of a food may be improved compared with the food not utilizing at least one component selected from the components (A) to (C) (e.g., cheese flavor can be imparted to the food). In one embodiment, in particular, by using the components (A) to (C) in combination, flavor of a food may be improved compared with the food utilizing the component (A) alone (e.g., cheese flavor may be imparted to the food). Measurement and comparison of flavor of foods (e.g., cheese flavor of foods) may be performed by, for example, sensory evaluation conducted by a panel of professional experts.

Flavor (e.g., cheese flavor) may be classified into, for example, those of initial taste, mid-taste, and aftertaste. The flavor of "initial taste", "mid-taste", and "aftertaste" refers to, in the case of liquids (liquid foods), flavor sensed from 0 to 1 second, 1 to 3 seconds, and 3 to 5 seconds, respectively, after drinking (after the foods are placed in the mouth). For solids (solid foods), the flavor of "initial taste", "mid-taste", and "aftertaste" refers to flavor sensed from 0 to 4 seconds, 4 to 10 seconds, and 10 to 15 seconds, respectively, after eating (after the foods are placed in the mouth). In the present invention, "solid" refers to a form other than liquid and includes pastes, gels, etc. By utilizing the active ingredient, for example, flavor of initial taste, flavor of mid-taste, flavor of aftertaste, or a combination thereof may be improved. That is, specifically, by utilizing the active ingredient, for example, cheese flavor of initial taste, cheese flavor of mid-taste, cheese flavor of aftertaste, or a combination thereof may be improved.

The carboxylic acid is selected from the group consisting of 2-methylbutyric acid, 3-methylbutyric acid, isobutyric acid, and a combination thereof. The combination is not particularly limited. Examples of the combination include combination of 2-methylbutyric acid and 3-methylbutyric acid, combination of 3-methylbutyric acid and isobutyric acid, combination of 2-methylbutyric acid and isobutyric acid, and combination of 2-methylbutyric acid, 3-methylbutyric acid, and isobutyric acid. Examples of 2-methylbutyric acid include (S)-2-methylbutyric acid and (R)-2-methylbutyric acid. As 2-methylbutyric acid, in particular, (S)-2-methylbutyric acid can be mentioned. As the carboxylic acid, at least 2-methylbutyric acid may be selected. As the carboxylic acid, for example, at least 2-methylbutyric acid may be selected, and further, 3-methylbutyric acid and/or isobutyric acid may also be selected. That is, the carboxylic acid may be, for example, 2-methylbutyric acid or a combination including it, in other words, it may contain 2-methylbutyric acid. As the carboxylic acid, for example, at least (S)-2-methylbutyric acid may be selected. That is, the carboxylic acid may be, for example, (S)-2-methylbutyric acid or a combination containing it, and in other words, it may contain (S)-2-methylbutyric acid. Also in other words, as 2-methylbutyric acid, for example, at least (S)-2-methylbutyric acid may be selected. As 2-methylbutyric acid, for example, at least (S)-2-methylbutyric acid may be selected, and (R)-2-methylbutyric acid may further be selected. That is, 2-methylbutyric acid may be, for example, (S)-2-methylbutyric acid or a combination containing it, and in other words, it may contain (S)-2-methylbutyric acid.

Each component of the active ingredient may be a commercially available product or one obtained by appropriate production. The production method for each component of the active ingredient is not particularly limited. Each component of the active ingredient may be produced by, for example, chemical synthesis, enzymatic reaction, fermentation, extraction, or a combination thereof. Specifically, each component of the active ingredient may be produced by, for example, utilizing a *Bacillus* bacterium as described below. Each component of the active ingredient may be purified to a desired degree or may not be purified. That is, each component of the active ingredient may be a purified product or a material containing the component of the active ingredient. Examples of such a material containing each component of the active ingredient include fermentation products such as culture broths, bacterial cells, culture supernatants, and processed products thereof, obtained by culturing a microorganism having an ability to produce that component of the active ingredient. Examples of the processed products include products obtained by subjecting materials such as the aforementioned fermentation products to such treatments as concentration, dilution, drying, fractionation, extraction, and purification. As each component of the active ingredient, materials containing that component of the active ingredient at a content of 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher may be used.

The carboxylic acid may be used as a free acid or a salt, or may be produced as a combination thereof. That is, unless especially specified, the term "carboxylic acid" may mean a free carboxylic acid, a salt thereof, or a combination thereof. For the salt of the carboxylic acid, the description regarding salts for the acidic group of the carboxylic acid precursor described later may be applied.

When using a material containing a component of the active ingredient, the amount of that component (e.g., content (concentration) or usage amount) shall be calculated based on the amount of that component of the active ingredient itself in the material. Further, when a component of the active ingredient forms a salt, the amount of that component of the active ingredient (e.g., content (concentration) or usage amount) shall be calculated based on the mass of the equimolar amount of that component in a free form.

### <1-2> Method for producing active ingredient

Hereafter, a method for producing the active ingredient using a bacterium of the genus *Bacillus* will be explained. That is, the active ingredient may be produced by this production method. This production method is also referred to as "the production method of the present invention".

The *Bacillus* bacterium may have one, two, or all three of carboxylic acid-producing ability, diacetyl-producing ability, and acetoin-producing ability. The *Bacillus* bacterium may have, in particular, all of the carboxylic acid-producing ability, diacetyl-producing ability, and acetoin-producing ability. A *Bacillus* bacterium having an ability to produce a carboxylic acid is also referred to as "carboxylic acid-producing bacterium". A *Bacillus* bacterium having an ability to produce diacetyl is also referred to as "diacetyl-producing bacterium". A *Bacillus* bacterium having an ability to produce acetoin is also referred to as "acetoin-producing bacterium". For example, a *Bacillus* bacterium having all of the carboxylic acid-producing ability, diacetyl-producing ability, and acetoin-producing ability is a carboxylic acid-producing bacterium, a diacetyl-producing bacterium, and an acetoin-producing bacterium.

The carboxylic acid-producing ability, diacetyl-producing ability, and acetoin-producing ability are also collectively referred to as "active ingredient-producing ability". A carboxylic acid-producing bacterium, diacetyl-producing bacterium, and acetoin-producing bacterium are also collectively referred to as "active ingredient-producing bacterium". Such an active ingredient-producing bacterium has the corresponding active ingredient-producing ability (i.e., a carboxylic acid-producing ability for carboxylic acid-producing bacterium, a diacetyl-producing ability for diacetyl-producing bacterium, and an acetoin-producing ability for acetoin-producing bacterium).

By the production method of the present invention, for example, each component of the active ingredient may be individually produced. Further, by the production method of the present invention, for example, two or all three types of the components of the active ingredient may be produced together. By the production method of the present invention, in particular, all the components of the active ingredient may be produced together.

The components of the active ingredient can be produced by culturing a corresponding active ingredient-producing bacterium (i.e., carboxylic acid-producing bacterium for carboxylic acid, diacetyl-producing bacterium for diacetyl, and acetoin-producing bacterium for acetoin) in a culture medium. However, when producing at least a carboxylic acid (i.e., when producing a carboxylic acid alone or in combination with diacetyl and/or acetoin), a medium containing a precursor of the carboxylic acid is used. Further, when producing at least diacetyl (i.e., when producing diacetyl alone or in combination with a carboxylic acid and/or acetoin), a medium containing a sugar is used. Furthermore, when producing at least acetoin (i.e., when producing acetoin alone or in combination with a carboxylic acid and/or diacetyl), a medium containing a sugar is used.

That is, the production method of the present invention is a method for producing the active ingredient, comprising
culturing a corresponding active ingredient-producing bacterium in a culture medium to obtain a culture containing the active ingredient.

For example, the component (A) (carboxylic acid) can be produced by culturing a carboxylic acid-producing bacterium in a culture medium containing a precursor of the carboxylic acid.

That is, one embodiment of the production method of the present invention is a method for producing a carboxylic acid, comprising
the step of culturing a carboxylic acid-producing bacterium in a culture medium containing a precursor of the carboxylic acid to obtain a culture containing the carboxylic acid, wherein
the carboxylic acid is selected from the group consisting of 2-methylbutyric acid, 3-methylbutyric acid, isobutyric acid, and a combination thereof.

Further, for example, the component (B) (diacetyl) can be produced by culturing a diacetyl-producing bacterium in a medium containing a sugar.

That is, one embodiment of the production method of the present invention is a method for producing diacetyl, comprising
the step of culturing a diacetyl-producing bacterium in a medium containing a sugar to obtain a culture containing diacetyl.

Further, for example, the component (C) (acetoin) can be produced by culturing an acetoin-producing bacterium in a medium containing a sugar.

That is, one embodiment of the production method of the present invention is a method for producing acetoin, comprising
the step of culturing an acetoin-producing bacterium in a medium containing a sugar to obtain a culture containing acetoin.

Furthermore, for example, the components (A), (B), and (C) (carboxylic acid, diacetyl, and acetoin) can be collectively produced by culturing a *Bacillus* bacterium having a carboxylic acid-producing ability, a diacetyl-producing ability, and an acetoin-producing ability in a medium containing a precursor of the carboxylic acid and a sugar.

That is, one embodiment of the production method of the present invention is a method for producing a carboxylic acid, diacetyl, and acetoin, comprising
the step of culturing a *Bacillus* bacterium having a carboxylic acid-producing ability, a diacetyl-producing ability, and an acetoin-producing ability in a medium containing a precursor of the carboxylic acid and a sugar to obtain a culture containing the carboxylic acid, diacetyl, and acetoin, wherein
the carboxylic acid is selected from the group consisting of 2-methylbutyric acid, 3-methylbutyric acid, isobutyric acid, and a combination thereof.

The term "bacterium having a carboxylic acid-producing ability" means a bacterium that has an ability to, when cultured in a medium containing a precursor of a carboxylic acid, produce the carboxylic acid and accumulate it in the medium and/or bacterial cells. The bacterium having a carboxylic acid-producing ability may at least accumulate a carboxylic acid in the medium. The bacterium having a carboxylic acid-producing ability may accumulate a carboxylic acid in the medium at a concentration of, for example, 100 ppm (w/w) or higher, 300 ppm (w/w) or higher, 500 ppm (w/w) or higher, 1,000 ppm (w/w) or higher, 2,000 ppm (w/w) or higher, 3,000 ppm (w/w) or higher, 4,000 ppm (w/w) or higher, 5,000 ppm (w/w) or higher, 6,000 ppm (w/w) or higher, 7,000 ppm (w/w) or higher, or 8,000 ppm (w/w) or higher. The bacterium having a carboxylic acid-producing ability may accumulate a carboxylic acid at a concentration of, for example, 50,000 ppm (w/w) or lower, 20,000 ppm (w/w) or lower, or 10,000 ppm (w/w) or lower in the culture medium. When the carboxylic acid-producing bacterium produces two or more types of carboxylic acids, it may accumulate these carboxylic acids in the medium independently or collectively at a concentration in any of the ranges exemplified above. The carboxylic acid-producing bacterium may accumulate, for example, 2-methylbutyric acid in the medium at a concentration in any of the ranges exemplified above. When two or more types of carboxylic acids are accumulated in the medium, the expression that "concentration of carboxylic acid accumulated in the medium" shall mean the total concentration of those two or more types of carboxylic acids accumulated in the medium, unless especially specified.

The term "bacterium having a diacetyl-producing ability" means a bacterium that has an ability to, when cultured in a medium containing a sugar, produce diacetyl and accumulate it in the medium and/or within the bacterial cells. The bacterium having a diacetyl-producing ability may accumulate diacetyl at least in the medium. The bacterium having a diacetyl-producing ability may accumulate diacetyl in the medium at a concentration of, for example, 50 ppm (w/w) or higher, 100 ppm (w/w) or higher, 200 ppm (w/w) or higher, 300 ppm (w/w) or higher, 400 ppm (w/w) or higher, or 500 ppm (w/w) or higher. The bacterium having a diacetyl-producing ability may accumulate diacetyl in the medium at a concentration of, for example, 5,000 ppm (w/w) or lower, 2,000 ppm (w/w) or lower, or 1,000 ppm (w/w) or lower.

The term "bacterium having an acetoin-producing ability" means a bacterium that has an ability to, when cultured in a medium containing a sugar, produce acetoin and accumulate it in the medium and/or within the bacterial cells. The bacterium having an acetoin-producing ability may accumulate acetoin at least in the medium. The bacterium having an acetoin-producing ability may accumulate acetoin in the medium at a concentration of, for example, 500 ppm (w/w) or higher, 1,000 ppm (w/w) or higher, 2,000 ppm (w/w) or higher, 3,000 ppm (w/w) or higher, 4,000 ppm (w/w) or higher, 5,000 ppm (w/w) or higher, 6,000 ppm (w/w) or higher, 7,000 ppm (w/w) or higher, 8,000 ppm (w/w) or higher, 9,000 ppm (w/w) or higher, or 10,000 ppm (w/w) or higher in the medium. The bacterium having an acetoin-producing ability may accumulate acetoin in the medium at a concentration of, for example, 50,000 ppm (w/w) or lower, 20,000 ppm (w/w) or lower, or 10,000 ppm (w/w) or lower.

The carboxylic acid is as described above. As the carboxylic acid, for example, at least 2-methylbutyric acid may be produced. As the carboxylic acid, for example, at least 2-methylbutyric acid may be produced, and further, 3-methylbutyric acid and/or isobutyric acid may also be produced. As the carboxylic acid, for example, at least (S)-2-methylbutyric acid may be produced. In other words, as 2-methylbutyric acid, for example, at least (S)-2-methylbutyric acid may be produced. As 2-methylbutyric acid, for example, at least (S)-2-methylbutyric acid may be produced, and further, (R)-2-methylbutyric acid may also be produced.

The precursor of the carboxylic acid is selected according to the type of the carboxylic acid. The precursor of the carboxylic acid is also simply referred to as "precursor".

Isoleucine (Ile) is an example of the precursor for producing 2-methylbutyric acid. In other words, the precursor of 2-methylbutyric acid may be isoleucine. Further, 2-methylbutyric acid may be the carboxylic acid corresponding to isoleucine.

Leucine (Leu) is an example of the precursor for producing 3-methylbutyric acid. In other words, the precursor of 3-methylbutyric acid may be leucine. Further, 3-methylbutyric acid may be the carboxylic acid corresponding to leucine.

Examples of the precursor for producing isobutyric acid include isoleucine (Ile), leucine (Leu), and valine (Val). In other words, the precursor of isobutyric acid may be selected from the group consisting of isoleucine, leucine, valine, and a combination thereof. Further, isobutyric acid may be a carboxylic acid corresponding to isoleucine, leucine, valine, or a combination thereof. The combination is not particularly limited. Examples of the combination include combinations of isoleucine and leucine, leucine and valine, isoleucine and valine, and isoleucine, leucine, and valine. The combination may include, for example, at least valine. As the precursor for producing isobutyric acid, in particular, valine (Val) can be mentioned. In other words, the precursor of isobutyric acid may be, in particular, valine. Further, isobutyric acid may be, in particular, a carboxylic acid corresponding to valine.

As the precursors for producing (S)-2-methylbutyric acid and (R)-2-methylbutyric acid, L-isoleucine and D-isoleucine can be mentioned, respectively. In other words, the precursor of (S)-2-methylbutyric acid and the precursor of (R)-2-methylbutyric acid may be L-isoleucine and D-isoleucine, respectively. Further, (S)-2-methylbutyric acid and (R)-2-methylbutyric acid may be a carboxylic acid corresponding to L-isoleucine and a carboxylic acid corresponding to D-isoleucine, respectively.

Unless especially specified, the precursor may be in the D-form, L-form, or a combination thereof. The ratio of the precursor in the D-form to L-form in the combination is not particularly limited. The ratio of the precursor in the D-form or L-form in the combination may be, for example, 20 to 80%, 30 to 70%, 40 to 60%, or 45 to 55% in molar ratio. The precursor may be in, in particular, the L-form. If a precursor in the D-form is selected, it is sufficient to use the precursor in the D-form, and concurrent use of the precursor in the L-form is not precluded. Similarly, if a precursor in the L-form is selected, it is sufficient to use the L-form precursor, and concurrent use of the precursor in the D-form is not precluded.

The precursor may be a commercially available product or one obtained by appropriate production. The production method of the precursor is not particularly limited. The precursor may be produced by, for example, chemical synthesis, enzymatic reaction, fermentation, extraction, or a combination thereof. Specifically, the precursor may be produced by, for example, culturing a microorganism having an ability to produce the precursor and recovering the precursor from the medium or the bacterial cells. The precursor may be purified to a desired degree or may not be purified. That is, as the precursor, a purified product or a material containing the precursor may be used. Examples of such a material containing the precursor include fermentation products such as culture, bacterial cells, and culture supernatant obtained by culturing a microorganism having an ability to produce the precursor, and processed products thereof. Examples of the processed products include materials such as the aforementioned fermentation products subjected to such processes as concentration, dilution, drying, fractionation, extraction, and purification. Examples of the material containing the precursor also include organic nitrogen sources such as yeast extract and peptone. As the precursor, a material containing the precursor at a content of 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher may be used.

The precursor may be used as a free compound, a salt, or a combination thereof. That is, unless especially specified, the term "precursor" may mean a precursor as a free compound, a salt thereof, or a combination thereof. The term "free compound" means a compound in a form that does not form a salt. The precursor may be in any form, such as an ion, at the time of use (e.g., during the culture process).

The salt is not particularly limited as long as the carboxylic acid is produced. As the salt, in particular, salts that are orally ingestible may be used. For example, salts for acidic groups such as carboxyl group include, specifically, ammonium salts, salts with alkali metal such as sodium and potassium, salts with alkaline earth metal such as calcium and magnesium, aluminum salts, zinc salts, and salts with organic amines such as triethylamine, ethanolamine, morpholine, pyrrolidine, piperidine, piperazine, and dicyclohexylamine, and salts with basic amino acid such as arginine and lysine. Further, specific examples of salts for basic groups such as amino group include salts with inorganic acids such as hydrochloric acid, sulfuric acid, phosphoric acid, nitric acid, and hydrobromic acid, salts with organic acids such as acetic acid, citric acid, benzoic acid, maleic acid, fumaric acid, tartaric acid, succinic acid, tannic acid, butyric acid, hibenzic acid, pamoic acid, enanthic acid, decanoic acid, theoclic acid, salicylic acid, lactic acid, oxalic acid, mandelic acid, malic acid, methylmalonic acid, and adipic acid, and salts with organic sulfonic acids such as methanesulfonic acid, benzenesulfonic acid, and p-toluenesulfonic acid. As the salt, one type of salt may be used, or two or more types of salts may be used in combination.

When using a material containing the precursor, the amount of the precursor (e.g., content (concentration) or usage amount) shall be calculated based on the amount of the precursor itself within the material containing the precursor, unless especially specified. Further, when the precursor forms a salt, the amount of the precursor (e.g., content (concentration) or usage amount) shall be calculated with the mass of the precursor in the free form in moles equivalent to that of the precursor in the form of the salt, unless especially specified.

The sugar is not particularly limited as long as it can be utilized by the active ingredient-producing bacterium. Examples of the sugar include monosaccharides and oligosaccharides such as glucose, fructose, sucrose, lactose, galactose, xylose, arabinose, maltose, isomaltose, and fructooligosaccharides. As the sugar, a purified product may be used, or a material containing a sugar may be used. As the sugar, a material having a sugar content of 1% (w/w) or higher, 5% (w/w) or higher, 10% (w/w) or higher, 30% (w/w) or higher, 50% (w/w) or higher, 70% (w/w) or higher, 90% (w/w) or higher, or 95% (w/w) or higher may be used. Examples of the material containing a sugar (e.g., monosaccharide or oligosaccharide) include invert sugars, molasses, starch hydrolysates, and plant biomass hydrolysates. Examples of molasses include cane molasses, beet molasses, high-test molasses, and citrus molasses. Examples of starch include cornstarch, tapioca starch, cassava starch, potato starch, and starches from various other grains. Examples of biomass include cellulose and hemicellulose. Plant biomass hydrolysates can be obtained by subjecting plant biomass to such processes as steam treatment, concentrated acid hydrolysis, diluted acid hydrolysis, enzymatic hydrolysis using cellulase or similar enzymes, and alkaline treatment. Since hemicellulose is generally more readily hydrolyzed than cellulose, hemicellulose in plant biomass may be pre-hydrolyzed to release pentoses, followed by hydrolysis of the cellulose to produce hexoses. Glucose is a particularly suitable sugar. As the sugar, commercially available products may be used, or sugars obtained by appropriate production may be used. As the sugar, one type of sugar may be used, or two or more types of sugars may be used in combination. For example, at least glucose may be used as the sugar. That is, as the sugar, for example, glucose alone may be used, or glucose may be used in combination with one or more other types of sugars. When using a material containing a sugar, the amount of the sugar (e.g., content (concentration) or usage amount) shall be calculated with the amount of the sugar itself in the material, unless especially specified.

The active ingredient-producing bacterium may be one inherently having an active ingredient-producing ability or may be one modified to have an active ingredient-producing ability. The active ingredient-producing bacterium can be obtained by, for example, conferring an active ingredient-producing ability to any *Bacillus* bacterium strain or by enhancing an active ingredient-producing ability of any *Bacillus* bacterium strain.

The method for imparting or enhancing the active ingredient-producing ability is not particularly limited. As the method for imparting or enhancing the active ingredient-producing ability, for example, known methods may be used. The impartation or enhancement of the active ingredient-producing ability may be carried out by, for example, mutagenesis methods or genetic engineering techniques.

Examples of the method for imparting or enhancing a carboxylic acid-producing ability include, for example, a method of modifying a bacterium so that activity or activities of one or more types of enzymes selected from enzymes that catalyze conversion of a precursor to a carboxylic acid is increased. Such enzymes are not particularly limited, and include a branched-chain-amino-acid transaminase (EC 2.6.1.42), leucine dehydrogenase (EC 1.4.1.9), L-amino-acid oxidase (EC 1.4.3.2), phenylpyruvate decarboxylase (EC 4.1.1.43), and pyruvate decarboxylase (EC 4.1.1.1). Enzymatic activity can be increased by, for example, enhancing expression of a gene encoding the enzyme. Gene expression can be increased by, for example, increasing the copy number of gene. Gene expression can be increased by, for example, modifying an expression regulatory sequence such as promoter (e.g., replacing it with more potent one). Gene expression can be increased by, for example, obtaining a strain with increased expression of a target gene using a mutagenesis method. Further, enzymatic activity can be increased by, for example, modifying a bacterium to have a gene with a mutation that increases specific activity of enzyme. Such a mutation that increases specific activity of enzyme may be a mutation in a gene encoding the enzyme, or it may be a mutation in a gene other than the gene encoding the enzyme. Such a bacterium having a gene with a mutation that increases enzymatic activity can be obtained by, for example, acquiring a bacterium with such a mutation using a mutagenesis method. Methods for increasing enzymatic activity are specifically disclosed in, for example, WO2015/060391 and WO2018/030507.

The active ingredient-producing bacterium may be one that inherently has an active ingredient-producing ability, or one that has been modified to have an active ingredient-producing ability by a method other than genetic engineering techniques (e.g., mutagenesis method). The active ingredient-producing bacterium may further be, in particular, one that inherently has an active ingredient-producing ability. The active ingredient-producing bacterium may further be, in particular, one that inherently has a carboxylic acid-producing ability, a diacetyl-producing ability, and an acetoin-producing ability.

Examples of *Bacillus* bacteria that can be used as the active ingredient-producing bacterium or as a parent strain for constructing it include *Bacillus subtilis*, *Bacillus amyloliquefaciens*, *Bacillus pumilus*, *Bacillus licheniformis*, *Bacillus megaterium*, *Bacillus brevis*, *Bacillus polymixa*, *Bacillus stearothermophilus*, and *Bacillus velezensis*. As the *Bacillus* bacteria, in particular, *Bacillus subtilis* and *Bacillus amyloliquefaciens* can be mentioned. As the *Bacillus* bacteria, *Bacillus subtilis* is especially noteworthy. Specific examples of *Bacillus subtilis* strains include 168 Marburg strain (ATCC 6051), PY79 strain (Plasmid, 1984, 12, 1-9), and IAM1114 strain (JCM20057). Specific examples of *Bacillus amyloliquefaciens* strains include F strain (ATCC 23350), T strain (ATCC 23842), N strain (ATCC 23845), AJ11708 strain (NITE BP-02609), and FZB42 strain (DSM 23117).

Various strains of *Bacillus* bacteria can be obtained from, for example, the American Type Culture Collection (Address: 12301 Parklawn Drive, Rockville, Maryland 20852 P.O. Box 1549, Manassas, VA 20108, United States of America). Each strain is assigned a corresponding registration number, which can be used to obtain the strain (refer to http://www.atcc.org/). The registration numbers for respective strains are listed in the catalog of American Type Culture Collection. Further, various strains of *Bacillus* bacteria can be obtained from, for example, the depositories where the strains have been deposited.

The step of culturing the active ingredient-producing bacterium in a medium to obtain a culture containing the active ingredient is also referred to as the "culture step". One embodiment of the culture step is a step of culturing a carboxylic acid-producing bacterium in a medium containing a precursor to obtain a culture containing a carboxylic acid. One embodiment of the culture step is a step of culturing a diacetyl-producing bacterium in a medium containing a sugar to obtain a culture containing diacetyl. One embodiment of the culture step is a step of culturing an acetoin-producing bacterium in a medium containing a sugar to obtain a culture containing acetoin. One embodiment of the culture step is a step of culturing an active ingredient-producing bacterium having a carboxylic acid-producing ability, diacetyl-producing ability, and acetoin-producing ability in a medium containing a precursor and a sugar to obtain a culture containing a carboxylic acid, diacetyl, and acetoin. The culture obtained by the culture step is also referred to as "fermentation broth" or "fermentation product".

The production method of the present invention for collectively producing all the components of the active ingredient will be described below. That is, unless especially specified, the term "active ingredient" in the following description for the production method of the present invention means all the components of the active ingredient (carboxylic acid, diacetyl, and acetoin). Further, unless especially specified, the term "active ingredient-producing bacterium" in the following description for the production method of the present invention means an active ingredient-producing bacterium having a carboxylic acid-producing ability, diacetyl-producing ability, and acetoin-producing ability.

The description for the production method of the present invention described below is applicable not only when producing all the components of the active ingredient together. When applying the description for the production method of the present invention described below to cases other than producing all the components of the active ingredient together, the terms "active ingredient" shall be read as component of the active ingredient to be produced, "active ingredient-producing bacterium" shall be read as active ingredient-producing bacterium corresponding to the active ingredient to be produced, "medium containing a precursor" shall be read as "medium containing a precursor as needed," and "medium containing a sugar" shall be read as "medium containing a sugar as needed". The expression that "the medium contains a precursor as needed" means that the medium contains a precursor when implementing the production method of the present invention in order to produce at least a carboxylic acid, but otherwise the medium may or may not contain a precursor. The expression that "the medium contains a sugar as needed" means that the medium contains a sugar at least when implementing the production method of the present invention in order to produce diacetyl or acetoin, but otherwise the medium may or may not contain a sugar. When the medium does not contain a sugar, it is preferable to utilize a component that can substitute for the carbon source, such as carbon sources other than sugar or organic nitrogen sources, as appropriate.

The medium to be used is not particularly limited as long as it contains the precursor and sugar, allows the active ingredient-producing bacterium to proliferate, and enables production of the active ingredient. As the medium, for example, conventional media used for culturing bacteria such as *Bacillus* bacteria, supplemented with the precursor and sugar, can be used. As the medium, for example, a medium containing components selected from a nitrogen source, phosphate source, sulfur source, and various other organic and inorganic components as needed, in addition to the precursor and sugar, can be used. The types and concentrations of the medium components may be appropriately set according to various conditions, such as the type of the active ingredient-producing bacterium to be used.

Specific examples of nitrogen source include, for example, ammonium salts such as ammonium sulfate, ammonium chloride, and ammonium phosphate; organic nitrogen sources such as peptone, yeast extract, meat extract, and hydrolyzed vegetable protein (HVP; e.g., hydrolyzed soy protein, soy sauce, and pea sauce); ammonia; and urea. Ammonia gas or aqueous ammonia used for pH adjustment may also be utilized as a nitrogen source. Further, as described in "Principles of Fermentation Technology, Gakkai Shuppan Center, 1988", the medium may be reused. One type of nitrogen source may be used, or two or more types of nitrogen sources may be used in combination.

Specific examples of phosphate source include, for example, phosphates such as potassium dihydrogen phosphate and dipotassium hydrogen phosphate, and phosphate polymers such as pyrophosphate. One type of phosphate source may be used, or two or more types of phosphate sources may be used in combination.

Specific examples of sulfur source include, for example, inorganic sulfur compounds such as sulfates, thiosulfates, and sulfites, and sulfur-containing amino acids such as cysteine, cystine, and glutathione. One type of sulfur source may be used, or two or more types of sulfur sources may be used in combination.

Specific examples of other various organic and inorganic components include, for example, inorganic salts such as sodium chloride and potassium chloride; trace metals such as iron, manganese, magnesium, and calcium; vitamins such as vitamin B1, vitamin B2, vitamin B6, nicotinic acid, nicotinamide, vitamin B12, biotin, and folic acid; amino acids; nucleic acids; organic components containing these, such as peptone, casamino acid, yeast extracts, and hydrolyzed vegetable proteins (HVP; e.g., hydrolyzed soy protein, soy sauce, and pea sauce), etc. Specific examples of other various organic and inorganic components also include defoamer, osmotic pressure regulator for the medium, and osmotic compensator. Examples of defoamer include silicone-based defoamers (oil-type, solution-type, oil compound-type, emulsion-type, self-emulsifying-type, etc.), alcohol-based defoamers, oil-based defoamers, polyether-based defoamers, and vegetable oils (cotton seed oil, linseed oil, soybean oil, olive oil, castor oil, coconut oil, etc.). Any form of defoamer, including liquid, paste, solid, powder, emulsion, or wax, can be employed. Examples of osmotic pressure regulator for the medium include salts such as sodium chloride and potassium chloride, and polysaccharides that cannot be utilized by microorganisms (e.g., sorbitol, dextrin etc.). Examples of osmotic compensator include potassium ions, betaine (glycine betaine), molasses (especially beet molasses), glutamic acid, and trehalose. Additionally, polymers selected from the group consisting of water-soluble cellulose derivatives, water-soluble polyvinyl compounds, polar organic solvent-soluble polyvinyl compounds, water-soluble starch derivatives, alginates, and polyacrylates may also be added to the medium. For these other various organic and inorganic components, a single type of component may be used, or two or more types of components may be used in combination.

Further, when using an auxotrophic mutant strain that requires an amino acid or the like for growth thereof, it is preferable to supplement the medium with the required nutrient.

The culture conditions are not particularly limited as long as the active ingredient-producing bacterium can proliferate and the active ingredient is produced, except that a medium containing the precursor and sugar is used. The culture can be carried out under conventional conditions used for culturing bacteria such as *Bacillus* bacteria. The culture conditions may be appropriately set according to various conditions, such as the type of the active ingredient-producing bacterium to be used.

The culture may be performed by using a liquid medium (i.e., by liquid culture). As the method for liquid culture, for example, the methods described in "Biotechnology Textbook Series 13: Culture Engineering, Toshiomi Yoshida, Corona Publishing Co., Ltd., 1998" can be used. Specifically, for liquid culture, for example, surface culture, submerged culture, membrane-separated culture (using dialysis membranes, hollow fibers, etc.), immobilized microbial culture etc. can be utilized. Further, culture apparatus such as aerated stirred-tank culture apparatus, airlift culture apparatus, packed-bed culture apparatus, and fluidized-bed culture apparatus can be used. For the culture, the methods described in "Principles of Fermentation Technology, Gakkai Shuppan Center, 1988" can be utilized. The culture may be conducted as seed culture and main culture, which are separately performed. It is sufficient that the active ingredient is produced in the main culture. It is sufficient that both the precursor and sugar are contained in the medium during the main culture. That is, when the culture is conducted separately as seed culture and main culture, the "culture step" refers to the step of culturing the active ingredient-producing bacterium in the medium during the main culture, unless especially specified. The culture conditions for the seed culture and the main culture may be the same or different. It is sufficient that at least the main culture is performed by using a liquid medium. For example, the active ingredient-producing bacterium cultured on a solid medium such as agar medium may be directly inoculated into a liquid medium, or the active ingredient-producing bacterium seed-cultured in a liquid medium may be inoculated into a liquid medium for the main culture. The amount of the active ingredient-producing bacterium present in the medium at the start of the culture is not particularly limited. The main culture may be performed by, for example, inoculating 1 to 50% (v/v) of the seed culture broth to the medium for the main culture. Further, the seed culture step may include two or more seed culture steps, for example, to obtain a bacterial cell amount necessary for the main culture step. Furthermore, the seed culture broth may be inoculated only at the start of the main culture, or additionally inoculated during the main culture in addition to the inoculation at the start of the main culture.

The culture may be performed as batch culture, fed-batch culture, continuous culture, or a combination thereof. Examples of the combination include culture in which two or more stages of fed-batch culture are connected in series and culture in which two or more stages of continuous culture are connected in series. The medium used at the start of the culture is also referred to as "initial medium". The medium supplied to the culture system (e.g., fermenter) during fed-batch culture or continuous culture is also referred to as "feed medium". Further, supplying feed medium to the culture system during fed-batch culture or continuous culture is also referred to as "feeding". When the culture is performed as separate seed culture and main culture, both seed culture and main culture may be performed, for example, as batch culture. It is also possible to perform, for example, seed culture as batch culture, and main culture as fed-batch culture or continuous culture. Alternatively, for example, the seed culture may be performed as fed-batch culture, and the main culture may be performed as batch culture. The feed medium may be supplied, for example, from a location not in contact with the liquid surface of the medium at the top of the culture tank, from a location within the medium such as the middle or bottom of the culture tank, or from both the top and middle of the culture tank. A method for supplying feed medium from a location within the medium is disclosed in, for example, Japanese Patent No. 6097869.

The respective medium components may be contained in the initial medium, feed medium, or both. The types of components contained in the initial medium may be the same as or different from the types of components contained in the feed medium. Further, the concentration of each component contained in the initial medium may be the same as or different from the concentration of that component contained in the feed medium. Further, two or more types of feed media differing in the types and/or concentrations of the components they contain may be used. For example, when feeding is intermittently performed multiple times, the types and/or concentrations of the components contained in the feed medium for each time may be the same or different.

The medium may or may not be sterilized. Sterilization of the medium may be performed, for example, to prevent microbial contamination. Sterilization of the medium may be referred to as "sterilization" or "sanitization". Methods for sterilizing the medium include sterilization under high temperature and high pressure conditions, sterilization by UV irradiation, and sterilization using a filter or membrane. Sterilization of the medium may be performed batchwise or continuously. Examples of the method for sterilization performed batchwise under high temperature and high pressure conditions include, for example, autoclave sterilization and batch sterilization performed within a culture tank. Further, examples of the method of continuously performing sterilization under high temperature and high pressure conditions include continuous sterilization using a plate heat exchanger. Furthermore, sterilization of the sugar may be performed simultaneously with sterilization of the other medium components or separately from sterilization of the other components. Preferably, the sugar and the other components may be sterilized separately.

The culture may be performed, for example, under aerobic conditions using a liquid medium. The term "aerobic conditions" may mean conditions where the dissolved oxygen concentration in the medium is 0.18 ppm or higher, 0.33 ppm or higher, or 1.5 ppm or higher. The dissolved oxygen concentration can be measured by using a sensor such as PL electrode or DO electrode. Culture under aerobic conditions can be performed by, for example, aerated culture or shaking culture. The pH of the medium can be, for example, 3 to 10, preferably 4.0 to 9.5. During the culture, the medium pH can be adjusted as necessary. The medium pH can be adjusted by using various alkaline or acidic substances, such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 40°C, preferably 25 to 37°C, more preferably 28 to 30°C. The culture period may be, for example, 10 to 120 hours. The culture may be continued, for example, until the sugar in the medium is consumed or until the activity of the active ingredient-producing bacterium is lost.

The contents of the medium components such as the precursor and sugar in the medium are not particularly limited as long as the active ingredient-producing bacterium can proliferate and produce the active ingredient.

The content of the precursor in the medium may be, for example, 0.1% (w/w) or higher, 0.2% (w/w) or higher, 0.3% (w/w) or higher, 0.5% (w/w) or higher, 0.7% (w/w) or higher, 1% (w/w) or higher, 1.5% (w/w) or higher, 2% (w/w) or higher, 2.5% (w/w) or higher, 3% (w/w) or higher, 3.5% (w/w) or higher, 4% (w/w) or higher, or 4.5% (w/w) or higher, and may be 20% (w/w) or lower, 10% (w/w) or lower, 5% (w/w) or lower, 4.5% (w/w) or lower, 4% (w/w) or lower, 3.5% (w/w) or lower, 3% (w/w) or lower, 2.5% (w/w) or lower, 2% (w/w) or lower, 1.5% (w/w) or lower, 1% (w/w) or lower, 0.7% (w/w) or lower, 0.5% (w/w) or lower, 0.3% (w/w) or lower, or 0.2% (w/w) or lower, or may be in a range defined by any non-contradictory combination of these minimum and maximum contents. The content of the precursor in the medium may specifically be, for example, 0.1 to 0.2% (w/w), 0.2 to 0.3% (w/w), 0.3 to 0.5% (w/w), 0.5 to 0.7% (w/w), 0.7 to 1% (w/w), 1 to 1.5% (w/w), 1.5 to 2% (w/w), 2 to 2.5% (w/w), 2.5 to 3% (w/w), 3 to 3.5% (w/w), 3.5 to 4% (w/w), 4 to 4.5% (w/w), 4.5 to 5% (w/w), or 10 to 20% (w/w), or may be in a range defined by any non-contradictory combination of the minimum and maximum contents of these ranges. The content of the precursor in the medium may specifically be, for example, 0.1 to 20% (w/w), 0.2 to 10% (w/w), 0.3 to 4% (w/w), or 0.5 to 3% (w/w). When the medium contains two or more types of precursors, the contents of those two or more types of precursors in the medium may be set independently or as a total content within the ranges of the content of the precursor in the medium exemplified above. Further, when the medium contains two or more types of precursors, "the content of the precursor in the medium" shall mean the total content of those two or more types of precursors in the medium, unless especially specified.

When the medium contains two or more types of precursors, the contents of these two or more types of precursors in the medium may or may not be the same. When the medium contains first and second precursors, the content of the second precursor in the medium relative to 100 parts by weight of the first precursor contained in the medium may be, for example, 0.1 parts by weight or more, 0.2 parts by weight or more, 0.5 parts by weight or more, 1 part by weight or more, 2 parts by weight or more, 5 parts by weight or more, 10 parts by weight or more, 20 parts by weight or more, 30 parts by weight or more, 50 parts by weight or more, or 70 parts by weight or more, and may be 100 parts by weight or less, 70 parts by weight or less, 50 parts by weight or less, 30 parts by weight or less, 20 parts by weight or less, 10 parts by weight or less, 5 parts by weight or less, 2 parts by weight or less, 1 part by weight or less, 0.5 parts by weight or less, or 0.2 parts by weight or less, or may be in a range defined by any non-contradictory combination of these minimum and maximum contents. The content of the second precursor contained in the medium relative to 100 parts by weight of the first precursor contained in the medium may specifically be, for example, 0.1 to 0.2 parts by weight, 0.2 to 0.5 parts by weight, 0.5 to 1 part by weight, 1 to 2 parts by weight, 2 to 5 parts by weight, 5 to 10 parts by weight, 10 to 20 parts by weight, 20 to 30 parts by weight, 30 to 50 parts by weight, 50 to 70 parts by weight, or 70 to 100 parts by weight. The content of the second precursor contained in the medium relative to 100 parts by weight of the first precursor contained in the medium may specifically be, for example, 0.1 to 100 parts by weight, 0.5 to 50 parts by weight, or 2 to 20 parts by weight. The first precursor may be any precursor, but may be, for example, isoleucine. The first precursor may be, in particular, for example, L-isoleucine. The second precursor may be any precursor other than the first precursor, but may be, for example, leucine or valine. For example, when the medium contains isoleucine, leucine, and valine, the leucine and valine contents in the medium may be, each independently or in total, the amounts exemplified above relative to 100 parts by weight of isoleucine contained in the medium.

The content of the precursor in the medium may be set so that, for example, a desired production amount of the corresponding carboxylic acid can be obtained. The content of the precursor in the medium may be, for example, an amount of 1 time or more relative to the desired production amount of the corresponding carboxylic acid in molar ratio. The content of the precursor in the medium may be, for example, 1 time or more, 1.1 times or more, 1.2 times or more, 1.3 times or more, 1.5 times or more, 1.7 times or more, 2 times or more, 2.5 times or more, 3 times or more, 5 times or more, or 7 times or more, and may be 10 times or less, 7 times or less, 5 times or less, 3 times or less, 2.5 times or less, 2 times or less, 1.7 times or less, 1.5 times or less, 1.3 times or less, 1.2 times or less, or 1.1 times or less, or may be in a range defined by any non-contradictory combination of these minimum and maximum contents, relative to the desired production amount of the corresponding carboxylic acid in molar ratio. The content of the precursor in the medium may specifically be, for example, 1 to 1.1 times, 1.1 to 1.2 times, 1.2 to 1.3 times, 1.3 to 1.5 times, 1.5 to 1.7 times, 1.7 to 2 times, 2 to 2.5 times, 2.5 to 3 times, 3 to 5 times, 5 to 7 times, or 7 to 10 times the desired production amount of the corresponding carboxylic acid in molar ratio. The content of the precursor in the medium may specifically be, for example, 1 to 10 times, 1 to 7 times, or 1 to 5 times the desired production amount of the corresponding carboxylic acid in molar ratio.

The content of the sugar in the medium may be, for example, 0.1% (w/w) or higher, 0.7% (w/w) or higher, 1% (w/w) or higher, 2% (w/w) or higher, 3% (w/w) or higher, 5% (w/w) or higher, 7% (w/w) or higher, 10% (w/w) or higher, 15% (w/w) or higher, 20% (w/w) or higher, 25% (w/w) or higher, 30% (w/w) or higher, or 40% (w/w) or higher, and may be 50% (w/w) or lower, 40% (w/w) or lower, 30% (w/w) or lower, 25% (w/w) or lower, 20% (w/w) or lower, 15% (w/w) or lower, 10% (w/w) or lower, 7% (w/w) or lower, 5% (w/w) or lower, 3% (w/w) or lower, or 2% (w/w) or lower, or may be in a range defined by any non-contradictory combination of these minimum and maximum contents. The content of the sugar in the medium may specifically be, for example, 0.1 to 0.7% (w/w), 1 to 2% (w/w), 2 to 3% (w/w), 3 to 5% (w/w), 5 to 7% (w/w), 7 to 10% (w/w), 10 to 15% (w/w), 15 to 20% (w/w), 20 to 25% (w/w), 25 to 30% (w/w), 30 to 40% (w/w), or 40 to 50% (w/w). The content of the sugar in the medium may specifically be, for example, 1 to 50% (w/w), 1 to 30% (w/w), or 3 to 10% (w/w). The content of the sugar in the medium may be, for example, as high as possible without inhibiting the production of the active ingredient. Further, the sugar may be additionally added to the medium as appropriate. For example, the sugar may be added to the medium as consumption thereof occurs during fermentation. In fed-batch culture or continuous culture, the sugar supply amount may be such an amount that a sufficient condition (i.e., a condition that the sugar is supplied in an amount exceeding the sugar utilization amount (ability) of the active ingredient-producing bacterium) is attained, or a limiting condition (i.e., a condition that the sugar is supplied in an amount insufficient for the sugar utilization amount (ability) of the active ingredient-producing bacterium) is attained.

Any of the medium components such as the precursor and sugar may be contained in the medium throughout the entire culture period or only during a part of the culture period. That is, the expression that "the culture is performed in a medium containing a certain component" or "a certain component is contained in the medium during culture" is satisfied if the component is contained in the medium for at least a part of the culture period, and it is not required that the component be present in the medium for the entire culture period. Any of the medium components such as the precursor and sugar may be contained in the medium, for example, at the start of culture, or may be supplied to the medium after the start of culture. Further, any of the medium components such as the precursor and sugar may be contained in the medium at the start of culture and may also be further supplied to the medium after the start of culture (e.g., after consumption of the component).

Any of the medium components such as the precursor and sugar may be contained in the medium at any of the concentrations exemplified above throughout the entire culture period, or they may be contained in the medium at any of the concentrations exemplified above only during a part of the culture period. That is, for satisfying the condition expressed as "the culture is performed in a medium containing a certain component at a certain concentration", "a certain component is contained in the medium at a certain concentration during the culture", or "concentration of a certain component in the medium during the culture is a certain concentration", it is sufficient for the concentration of the component in the medium to be within the specified concentration range for at least a part of the culture period, and it is not required that the concentration of the component remain within that range throughout the entire culture period. The medium components such as the precursor and sugar may be contained in the medium at the concentrations exemplified above at the start of the culture, or they may be supplied to the medium after the start of the culture to achieve the concentrations exemplified above. Further, any of the medium components such as the precursor or sugar may be contained in the medium at the concentration exemplified above, for example, at the start of the culture and may also be further supplied to the medium after the start of the culture (e.g., after consumption of the component) to attain the concentrations exemplified above.

The length of the "part of the culture period" is not particularly limited as long as the active ingredient can be produced. The length of the "part of the culture period" can be appropriately set according to various conditions such as the types of medium components, the type of active ingredient-producing bacterium used, the length of the culture period, and the desired amount of the active ingredient to be produced. The "part of the culture period" may be a period corresponding to, for example, 10% or more, 20% or more, 30% or more, 40% or more, 50% or more, 60% or more, 70% or more, 80% or more, 90% or more, 95% or more, 97% or more, or 99% or more of the entire culture period. Further, the "part of the culture period" may also be a period of, for example, 10 hours or longer, 20 hours or longer, 40 hours or longer, 60 hours or longer, 80 hours or longer, 100 hours or longer, 120 hours or longer, or 150 hours or longer. The term "entire culture period" means the entire period of the main culture when the culture is performed as separate seed culture and main culture.

The contents of the medium components such as the precursor or sugar in the medium can be measured by, for example, known methods used for detecting or identifying compounds. Examples of such methods include, for example, HPLC, UPLC, LC/MS, GC/MS, and NMR. These methods can also be used to confirm generation of the active ingredient. These methods may be used each alone or in any suitable combination.

By culturing the active ingredient-producing bacterium as described above, the active ingredient is accumulated in the medium and/or within the bacterial cells, thereby yielding a culture (i.e., fermentation broth) containing the active ingredient. In one embodiment, 2-methylbutyric acid and 3-methylbutyric acid can be detected and quantified as a single component. In one embodiment, the amount ratio (e.g., weight ratio or molar ratio) of 2-methylbutyric acid to 3-methylbutyric acid contained in the culture may be conveniently considered to be the same as the amount ratio (e.g., weight ratio or molar ratio) of isoleucine to leucine consumed during the culture.

The carboxylic acid may be produced as a free acid, a salt, or a combination thereof. That is, unless especially specified, the term "carboxylic acid" may mean a free carboxylic acid, a salt thereof, or a combination thereof. For the salt of carboxylic acid, the description of salts for the acidic group in the precursor can apply.

The active ingredient may be produced (e.g., obtained and utilized) as a composition containing the active ingredient. That is, the active ingredient produced may be a composition containing the active ingredient. In one embodiment, the amount ratio (e.g., weight ratio or molar ratio) of 2-methylbutyric acid to 3-methylbutyric acid contained in such a composition may be conveniently considered to be the same as the amount ratio (e.g., weight ratio or molar ratio) of isoleucine to leucine consumed during the culture. The composition containing the active ingredient may be, for example, the composition of the present invention described later.

That is, the production method of the present invention may also be a method for producing a composition containing the active ingredient.

The production method of the present invention may specifically be a method for producing a composition containing a carboxylic acid, diacetyl, and acetoin, comprising
the step of culturing a *Bacillus* bacterium having a carboxylic acid-producing ability, a diacetyl-producing ability, and an acetoin-producing ability in a medium containing a precursor of the carboxylic acid and a sugar to obtain a culture containing the carboxylic acid, diacetyl, and acetoin, wherein
the carboxylic acid is selected from the group consisting of 2-methylbutyric acid, 3-methylbutyric acid, isobutyric acid, and a combination thereof.

The composition containing the active ingredient may consist of the active ingredient or may contain components other than the active ingredient. Such components other than the active ingredient are not particularly limited. The components other than the active ingredient can be selected as appropriate depending on various conditions such as the intended use of the active ingredient. The components other than the active ingredient may originate from, for example, a culture (i.e., fermentation broth) containing the active ingredient, or may be separately blended. Examples of the components other than the active ingredient include bacterial cells, medium components, moisture, and bacterial metabolic byproducts. Examples of the components other than the active ingredient also include components blended into foods or pharmaceuticals. Specific examples of such components blended into foods or pharmaceuticals include additives such as excipients.

The active ingredient may be produced (e.g., obtained and utilized) as, for example, a culture (i.e., fermentation broth) containing the active ingredient. That is, the produced active ingredient (e.g., a composition containing the active ingredient) may be, for example, a culture (i.e., fermentation broth) containing the active ingredient. Further, the active ingredient may be recovered from the culture (i.e., fermentation broth). That is, the production method of the present invention may further comprise a step of recovering the active ingredient from the culture (i.e., fermentation broth). The active ingredient may be recovered as an appropriate fraction containing the active ingredient. An example of such a fraction is culture supernatant. Such culture supernatant may be obtained by, for example, subjecting the culture to centrifugation. Further, the active ingredient may be further separated and purified from such a fraction as mentioned above. For example, the active ingredient may be recovered from culture supernatant obtained by separating the cells of the active ingredient-producing bacterium from the culture. Furthermore, if the active ingredient accumulates within the bacterial cells, the cells may be disrupted by, for example, ultrasonication, supernatant may be obtained by centrifugation or similar methods, and the active ingredient may be recovered from the supernatant. The recovery of the active ingredient can be performed by using known techniques employed for separating and purifying compounds. Examples of such techniques include ion exchange resin methods (Nagai, H. et al., Separation Science and Technology, 39(16), 3691-3710), precipitation methods, membrane separation methods (JP H09-164323 A, JP H09-173792 A), and crystallization methods (WO2008/078448, WO2008/078646). Further, a fraction containing the active ingredient (e.g., culture or culture supernatant) may be subjected to an appropriate treatment before use. Examples of such a treatment include concentration, drying, and heating. Concentration, drying, and heating can all be performed by, for example, using known methods. Examples of known drying methods include spray drying, freeze drying, drum drying, and vacuum drum drying. That is, the produced active ingredient (e.g., a composition containing the active ingredient) may be a culture of the active ingredient-producing bacterium, culture supernatant recovered from the culture, processed products thereof, or the active ingredient recovered from these. As the processed products, in particular, dried products (e.g., dried culture or dried culture supernatant) can be mentioned. Specific examples of the dried products include dried powders (e.g., dried powder of culture or dried powder of culture supernatant). The dried products (e.g., dried powders) may or may not undergo a treatment other than drying. The dried products (e.g., dried powders) may or may not contain components other than the active ingredient (e.g., additives such as excipients).

The recovered active ingredient may contain components other than the active ingredient, such as bacterial cells, medium components, moisture, and/or bacterial metabolic byproducts. The recovered active ingredient may contain, in particular, bacterial cells of the active ingredient-producing bacterium. The recovered active ingredient may be purified to a desired degree. The purity of the recovered active ingredient may be, for example, 50% (w/w) or higher, preferably 85% (w/w) or higher, more preferably 95% (w/w) or higher (JP 1214636 B, US 5,431,933 B2, US 4,956,471 B2, US 4,777,051 B2, US 4,946,654 B2, US 5,840,358 B2, US 6,238,714 B2, US2005/0025878).

The produced active ingredient may be utilized, for example, as is or in combination with components other than the active ingredient. Specifically, the produced active ingredient may be utilized, for example, as is or in combination with components other than the active ingredient, as a composition containing the active ingredient. Further, components other than the active ingredient may be incorporated during the production of the active ingredient (e.g., during processing a fraction containing the active ingredient). For example, additives such as excipients may be added during a treatment step such as drying.

Uses of the produced active ingredient (e.g., a composition containing the active ingredient) are not particularly limited. The produced active ingredient (e.g., a composition containing the active ingredient) can be used, for example, for uses of the composition of the present invention described below. Further, the produced active ingredient (e.g., a composition containing the active ingredient) can be used, for example, for uses of the method of the present invention described below.

### <2> Composition of the Present Invention

The composition of the present invention is a composition containing the active ingredient (i.e., carboxylic acid, diacetyl, and acetoin). The active ingredient may be produced by the production method of the present invention. That is, the composition of the present invention may be a composition containing the active ingredient (i.e., carboxylic acid, diacetyl, and acetoin), wherein the active ingredient has been produced by the production method of the present invention. When the active ingredient is produced as a composition containing the active ingredient in the production method of the present invention, the composition is an example of the composition of the present invention. That is, the composition of the present invention may be a composition containing the active ingredient (i.e., carboxylic acid), wherein the composition is produced by the production method of the present invention.

As described above, by utilizing the active ingredient, flavor of foods can be improved (e.g., cheese flavor can be imparted to foods). Therefore, the composition of the present invention may be, for example, a composition for improving flavor of foods (e.g., a composition for imparting cheese flavor to foods).

Further, by utilizing the active ingredient, foods with improved flavor (e.g., foods imparted with cheese flavor) can be produced. Therefore, the composition of the present invention may also be, for example, a composition for producing foods (specifically, for producing foods with improved flavor, such as foods imparted with cheese flavor).

The composition of the present invention may be, for example, a seasoning. Specifically, the composition of the present invention may be, for example, a seasoning for improving flavor of foods (e.g., seasoning for imparting cheese flavor to foods), and may also be a seasoning for producing foods (specifically, for producing foods with improved flavor such as foods imparted with cheese flavor).

The composition of the present invention may be used, for example, to improve flavor of foods or to produce foods in the manner described for the method of the present invention explained below.

The composition of the present invention can be produced by, for example, producing all the components of the active ingredient together by the production method of the present invention. The composition of the present invention can also be produced by, for example, producing the respective components of the active ingredient separately by the production method of the present invention and then combining them.

The composition of the present invention may contain one type of carboxylic acid or may contain two or more types of carboxylic acids as the component (A). The composition of the present invention containing two or more types of carboxylic acids can be produced by, for example, producing two or more types of carboxylic acids together by using the production method of the present invention (i.e., by implementing the production method of the present invention using a medium containing precursors of two or more types of carboxylic acids). Further, the composition of the present invention containing two or more types of carboxylic acids can also be produced by, for example, separately producing two or more types of carboxylic acids by the production method of the present invention and then combining them.

The composition of the present invention may consist of the active ingredient or may also contain components other than the active ingredient. Such components other than the active ingredient are not particularly limited. The components other than the active ingredient may be appropriately selected according to various conditions, such as the intended use of the active ingredient. The components other than the active ingredient may originate from, for example, a culture containing the active ingredient (i.e., fermentation broth) or may be separately blended. Examples of the components other than the active ingredient include bacterial cells, medium components, moisture, and bacterial metabolic byproducts. Examples of the components other than the active ingredient may also include components blended into foods or pharmaceuticals. Specific examples of the components blended into foods or pharmaceuticals include additives such as excipients.

The composition of the present invention may be, for example, suitably formulated. For formulating it, additives may be used as appropriate. Examples of such additives include excipients, binders, disintegrants, lubricants, stabilizers, flavoring agents and odorants, diluents, surfactants, and solvents. The additives may be selected as appropriate, for example, depending on various conditions such as the shape of the composition of the present invention.

The form of the composition of the present invention is not particularly limited. The composition of the present invention may be in any form, such as powder, flakes, tablets, paste, and liquid.

The contents and content ratios of the respective components in the composition of the present invention (i.e., the active ingredient and optionally other components) are not particularly limited as long as the desired effect, such as the flavor-improving effect, can be obtained. The contents and content ratios of the respective components in the composition of the present invention can be appropriately set depending on various conditions, such as the manner of use of the composition of the present invention.

The total content of the active ingredient in the composition of the present invention is higher than 0% (w/w) and 100% (w/w) or lower. The content of each component of the active ingredient in the composition of the present invention is higher than 0% (w/w) and lower than 100% (w/w).

The content of the carboxylic acid in the composition of the present invention may be, for example, 10 ppm (w/w) or higher, 50 ppm (w/w) or higher, 100 ppm (w/w) or higher, 500 ppm (w/w) or higher, 1,000 ppm (w/w) or higher, 5,000 ppm (w/w) or higher, 10,000 ppm (w/w) or higher, 20,000 ppm (w/w) or higher, or 50,000 ppm (w/w) or higher, and may be 90,000 ppm (w/w) or lower, 50,000 ppm (w/w) or lower, 20,000 ppm (w/w) or lower, 10,000 ppm (w/w) or lower, 5,000 ppm (w/w) or lower, 1,000 ppm (w/w) or lower, 500 ppm (w/w) or lower, 100 ppm (w/w) or lower, or 50 ppm (w/w) or lower, or may be in a range defined by any non-contradictory combination of these minimum and maximum contents. The content of the carboxylic acid in the composition of the present invention may specifically be, for example, 10 to 50 ppm (w/w), 50 to 100 ppm (w/w), 100 to 500 ppm (w/w), 500 to 1,000 ppm (w/w), 1,000 to 5,000 ppm (w/w), 5,000 to 10,000 ppm (w/w), 10,000 to 20,000 ppm (w/w), 20,000 to 50,000 ppm (w/w), or 50,000 to 90,000 ppm (w/w), or may be in a range defined by any non-contradictory combination of the minimum and maximum contents of these ranges. The content of the carboxylic acid in the composition of the present invention may specifically be, for example, 10 to 90,000 ppm (w/w), 50 to 50,000 ppm (w/w), or 100 to 20,000 ppm (w/w). When the composition of the present invention contains two or more types of carboxylic acids, the contents of those two or more types of carboxylic acids in the composition of the present invention may be set independently or in total within the ranges of carboxylic acid content in the composition of the present invention exemplified above (provided that the total content of those two or more types of carboxylic acids in the composition of the present invention is 100% (w/w) or lower). In one embodiment, for example, the content of 2-methylbutyric acid in the composition of the present invention may be 10 to 90,000 ppm (w/w), 50 to 50,000 ppm (w/w), or 100 to 20,000 ppm (w/w). In one embodiment, for example, the content of (S)-2-methylbutyric acid, in particular, in the composition of the present invention may be 10 to 90,000 ppm (w/w), 50 to 50,000 ppm (w/w), or 100 to 20,000 ppm (w/w). In one embodiment, for example, the total content of 2-methylbutyric acid and 3-methylbutyric acid in the composition of the present invention may be 10 to 90,000 ppm (w/w), 50 to 50,000 ppm (w/w), or 100 to 20,000 ppm (w/w). In one embodiment, for example, the contents of 3-methylbutyric acid and isobutyric acid in the composition of the present invention may each independently be 10 to 20,000 ppm (w/w), 10 to 5,000 ppm (w/w), or 10 to 1,000 ppm (w/w). When the composition of the present invention contains two or more types of carboxylic acids, "the content of the carboxylic acid in the composition of the present invention" means the total content of those two or more types of carboxylic acids in the composition of the present invention, unless especially specified.

When the composition of the present invention contains two or more types of carboxylic acids, the contents of those two or more types of carboxylic acids in the composition may or may not be the same. When the composition of the present invention contains first and second carboxylic acids, the content of the second carboxylic acid in the composition relative to 100 parts by weight of the first carboxylic acid contained in the composition may be, for example, 0.1 parts by weight or more, 0.2 parts by weight or more, 0.5 parts by weight or more, 1 part by weight or more, 2 parts by weight or more, 5 parts by weight or more, 10 parts by weight or more, 20 parts by weight or more, 30 parts by weight or more, 50 parts by weight or more, or 70 parts by weight or more, and may be 100 parts by weight or less, 70 parts by weight or less, 50 parts by weight or less, 30 parts by weight or less, 20 parts by weight or less, 10 parts by weight or less, 5 parts by weight or less, 2 parts by weight or less, 1 part by weight or less, 0.5 parts by weight or less, or 0.2 parts by weight or less, or may be in a range defined by any non-contradictory combination of these minimum and maximum contents. The content of the second carboxylic acid in the composition relative to 100 parts by weight of the first carboxylic acid contained in the composition may specifically be, for example, 0.1 to 0.2 parts by weight, 0.2 to 0.5 parts by weight, 0.5 to 1 part by weight, 1 to 2 parts by weight, 2 to 5 parts by weight, 5 to 10 parts by weight, 10 to 20 parts by weight, 20 to 30 parts by weight, 30 to 50 parts by weight, 50 to 70 parts by weight, or 70 to 100 parts by weight. The content of the second carboxylic acid in the composition relative to 100 parts by weight of the first carboxylic acid contained in the composition may specifically be, for example, 0.1 to 100 parts by weight, 0.5 to 50 parts by weight, or 2 to 20 parts by weight. The first carboxylic acid may be any carboxylic acid, but may be, for example, 2-methylbutyric acid. The first carboxylic acid may be, for example, (S)-2-methylbutyric acid, in particular. The second carboxylic acid may be any carboxylic acid other than the first carboxylic acid, but may be, for example, 3-methylbutyric acid or isobutyric acid. For example, when the composition of the present invention contains 2-methylbutyric acid, 3-methylbutyric acid, and isobutyric acid, the contents of 3-methylbutyric acid and isobutyric acid in the composition may, each independently or in total, be the amount exemplified above relative to 100 parts by weight of 2-methylbutyric acid contained in the composition. In one embodiment, when the composition of the present invention or the active ingredient contained therein is produced by the production method of the present invention, the amount ratio (e.g., weight ratio or molar ratio) of 2-methylbutyric acid to 3-methylbutyric acid contained in the composition of the present invention may be conveniently regarded to be equal to the amount ratio (e.g., weight ratio or molar ratio) of isoleucine to leucine consumed in the culture of the production method of the present invention.

Further, the content of the carboxylic acid in the composition of the present invention relative to 100 parts by weight of diacetyl contained in the composition may be, for example, 0.5 parts by weight or more, 1 part by weight or more, 2 parts by weight or more, 3 parts by weight or more, 4 parts by weight or more, 5 parts by weight or more, 6 parts by weight or more, 8 parts by weight or more, 10 parts by weight or more, 12 parts by weight or more, 15 parts by weight or more, or 20 parts by weight or less, and may be 15 parts by weight or less, 12 parts by weight or less, 10 parts by weight or less, 8 parts by weight or less, 6 parts by weight or less, 5 parts by weight or less, 4 parts by weight or less, 3 parts by weight or less, 2 parts by weight or less, or 1 part by weight or less, or may be in a range defined by any non-contradictory combination of these minimum and maximum contents. The content of the carboxylic acid in the composition of the present invention relative to 100 parts by weight of diacetyl contained in the composition may specifically be, for example, 0.5 to 1 part by weight, 1 to 2 parts by weight, 2 to 3 parts by weight, 3 to 4 parts by weight, 4 to 5 parts by weight, 5 to 6 parts by weight, 6 to 8 parts by weight, 8 to 10 parts by weight, 10 to 12 parts by weight, 12 to 15 parts by weight, or 15 to 20 parts by weight. The content of the carboxylic acid in the composition of the present invention relative to 100 parts by weight of diacetyl contained in the composition may specifically be, for example, 0.5 to 20 parts by weight, 1 to 15 parts by weight, or 2 to 12 parts by weight. The content of the carboxylic acid in the composition of the present invention relative to 100 parts by weight of diacetyl contained in the composition may specifically be, for example, 0.5 to 8 parts by weight, 1 to 6 parts by weight, or 2 to 4 parts by weight.

Further, the content of the carboxylic acid in the composition of the present invention relative to 100 parts by weight of acetoin contained in the composition may be, for example, 10 parts by weight or more, 20 parts by weight or more, 50 parts by weight or more, 70 parts by weight or more, 100 parts by weight or more, 150 parts by weight or more, 200 parts by weight or more, 300 parts by weight or more, 400 parts by weight or more, 500 parts by weight or more, or 700 parts by weight or more, and may be 1,000 parts by weight or less, 700 parts by weight or less, 500 parts by weight or less, 400 parts by weight or less, 300 parts by weight or less, 200 parts by weight or less, 150 parts by weight or less, 100 parts by weight or less, 70 parts by weight or less, 50 parts by weight or less, or 20 parts by weight or less, or may be in a range defined by any non-contradictory combination of these minimum and maximum contents. The content of the carboxylic acid in the composition of the present invention relative to 100 parts by weight of acetoin contained in the composition may specifically be, for example, 10 to 20 parts by weight, 20 to 50 parts by weight, 50 to 70 parts by weight, 70 to 100 parts by weight, 100 to 150 parts by weight, 150 to 200 parts by weight, 200 to 300 parts by weight, 300 to 400 parts by weight, 400 to 500 parts by weight, 500 to 700 parts by weight, or 700 to 1,000 parts by weight. The content of the carboxylic acid in the composition of the present invention relative to 100 parts by weight of acetoin contained in the composition may specifically be, for example, 10 to 1,000 parts by weight, 20 to 700 parts by weight, or 50 to 500 parts by weight. The content of the carboxylic acid in the composition of the present invention relative to 100 parts by weight of acetoin contained in the composition may specifically be, for example, 10 to 300 parts by weight, 20 to 200 parts by weight, or 50 to 150 parts by weight.

The content of diacetyl in the composition of the present invention may be 0.5 ppm (w/w) or higher, 1 ppm (w/w) or higher, 5 ppm (w/w) or higher, 10 ppm (w/w) or higher, 50 ppm (w/w) or higher, 100 ppm (w/w) or higher, 500 ppm (w/w) or higher, 1,000 ppm (w/w) or higher, or 2,000 ppm (w/w) or higher, and may be 5,000 ppm (w/w) or lower, 2,000 ppm (w/w) or lower, 1,000 ppm (w/w) or lower, 500 ppm (w/w) or lower, 100 ppm (w/w) or lower, 50 ppm (w/w) or lower, 10 ppm (w/w) or lower, 5 ppm (w/w) or lower, or 1 ppm (w/w) or lower, or may be in a range defined by any non-contradictory combination of these minimum and maximum contents. The content of diacetyl in the composition of the present invention may specifically be, for example, 0.5 to 1 ppm (w/w), 1 to 5 ppm (w/w), 5 to 10 ppm (w/w), 10 to 50 ppm (w/w), 50 to 100 ppm (w/w), 100 to 500 ppm (w/w), 500 to 1,000 ppm (w/w), 1,000 to 2,000 ppm (w/w), or 2,000 to 5,000 ppm (w/w). The content of diacetyl in the composition of the present invention may specifically be, for example, 0.5 to 5,000 ppm (w/w), 1 to 2,000 ppm (w/w), or 5 to 1,000 ppm (w/w).

The content of acetoin in the composition of the present invention may be, for example, 10 ppm (w/w) or higher, 50 ppm (w/w) or higher, 100 ppm (w/w) or higher, 500 ppm (w/w) or higher, 1,000 ppm (w/w) or higher, 5,000 ppm (w/w) or higher, 10,000 ppm (w/w) or higher, 20,000 ppm (w/w) or higher, or 50,000 ppm (w/w) or higher, and may be 90,000 ppm (w/w) or lower, 50,000 ppm (w/w) or lower, 20,000 ppm (w/w) or lower, 10,000 ppm (w/w) or lower, 5,000 ppm (w/w) or lower, 1,000 ppm (w/w) or lower, 500 ppm (w/w) or lower, 100 ppm (w/w) or lower, or 50 ppm (w/w) or lower, or may be in a range defined by any non-contradictory combination of these minimum and maximum contents. The content of acetoin in the composition of the present invention may specifically be, for example, 10 to 50 ppm (w/w), 50 to 100 ppm (w/w), 100 to 500 ppm (w/w), 500 to 1,000 ppm (w/w), 1,000 to 5,000 ppm (w/w), 5,000 to 10,000 ppm (w/w), 10,000 to 20,000 ppm (w/w), 20,000 to 50,000 ppm (w/w), or 50,000 to 90,000 ppm (w/w), or may be in a range defined by any non-contradictory combination of these minimum and maximum contents. The content of acetoin in the composition of the present invention may specifically be, for example, 10 to 90,000 ppm (w/w), 50 to 50,000 ppm (w/w), or 100 to 20,000 ppm (w/w).

The contents of the respective components (i.e., the active ingredient and optionally other components) in the composition of the present invention may be set, for example, to obtain the amounts of the respective components added in the method of the present invention described below.

The respective components contained in the composition of the present invention (i.e., the active ingredient and optionally other components) may be mixed together and contained in the composition of the present invention, or may be contained in the composition of the present invention separately or separately in any combination thereof. For example, the composition of the present invention may be provided as a set of components packaged separately. In such a case, the components included in the set may be used together as appropriate at the time of use.

### <3> Method of the present invention

The method of the present invention is a method comprising the step of utilizing the active ingredient (namely, carboxylic acid, diacetyl, and acetoin). The active ingredient may be one produced by the production method of the present invention. That is, the method of the present invention may be a method comprising the step of utilizing the active ingredient (namely, carboxylic acid, diacetyl, and acetoin), wherein the active ingredient has been produced by the production method of the present invention.

By the method of the present invention, specifically, by utilizing the active ingredient, flavor of foods can be improved (e.g., cheese flavor can be imparted to foods). Therefore, the method of the present invention may be implemented for improving flavor of foods (e.g., imparting cheese flavor to foods). That is, the method of the present invention may be, for example, a method for improving flavor of foods (e.g., a method for imparting cheese flavor to foods). This method is also referred to as "flavor-improving method of the present invention".

Further, by the method of the present invention, specifically by utilizing the active ingredient, foods with improved flavor (e.g., foods imparted with cheese flavor) can be produced. Therefore, the method of the present invention may be implemented for the production of foods (specifically, the production of foods with improved flavor such as foods imparted with cheese flavor). That is, the method of the present invention may be, for example, a method for producing foods (specifically, a method for producing foods with improved flavor such as foods imparted with cheese flavor). This method is also referred to as the "food production method of the present invention".

The active ingredient can be utilized to improve flavor or in food production by adding it to raw materials of foods during the food production. That is, one use of the active ingredient involves adding it to raw materials of foods. That is, the method of the present invention may specifically be a method for improving flavor of foods (e.g., a method for imparting cheese flavor to foods), comprising, for example, adding the active ingredient to raw materials of foods. Further, the method of the present invention may specifically be a method for producing foods (specifically, for producing foods with improved flavor such as foods imparted with cheese flavor), comprising, for example, adding the active ingredient to raw materials of foods. The term "blend" is also used instead of the term "add". The amounts of the respective components of the active ingredient added to the raw materials of foods in the method of the present invention may be amounts sufficient to achieve the contents of the respective components of the active ingredient in the composition of the present invention mentioned above.

The active ingredient may be utilized in the method of the present invention, for example, in the form of the composition of the present invention. That is, the "use of the active ingredient" also includes use of the composition of the present invention. For example, the "addition of the active ingredient" also includes addition of the composition of the present invention.

The food obtained by the method of the present invention is also referred to as the "food of the present invention". Specifically, the food of the present invention is a food with improved flavor (e.g., a food imparted with cheese flavor). Further, the food of the present invention is, in other words, a food to which the active ingredient has been added. Furthermore, the food of the present invention is, in other words, a food containing the active ingredient.

The improvement of flavor or production of the food may be carried out, for example, in the same manner as conventional food production, except for utilizing the active ingredient. That is, the improvement of flavor or production of the food may be carried out by using the same raw materials and under the same production conditions as those for conventional foods, except for utilizing the active ingredient. Further, such raw materials and production conditions of foods may be appropriately modified and utilized for the improvement of flavor or production of the food.

The type of the food is not particularly limited so long as the food is a food for which the desired effects, such as the flavor-improving effect, are desired to be exhibited. Examples of the food include foods containing cheese. Examples of the food also include foods not containing cheese. Typical examples of the food include foods containing cheese with reduced cheese content. The food also includes beverages. The food also includes seasonings. The food may be liquid or solid. Specific examples of the food include: beverages such as soft drinks, alcoholic beverages, and soups; rice dishes such as chicken rice, omelet rice, and paella; stewed dishes such as curry, beef stew, hayashi rice, and hashed beef; roux such as curry roux; sauces such as cheese sauce and meat sauce; pizza such as pizza Margherita; pasta such as carbonara and Bolognese; and confectioneries such as ice cream, yogurt, mousse, cake, and snack foods. The term "soft drink" may mean non-alcoholic beverages (beverages with an alcohol content of less than 1%) excluding milk and dairy products.

The form in which the food is provided is not particularly limited. The food may be provided, for example, in a form ready for immediate eating, or in a form requiring preparation before or during eating, such as concentrates or dried products. The food may also be provided in any container, such as retort pouches, paper cartons, plastic bottles such as PET bottles, metal cans such as steel cans and aluminum cans, or glass bottles. The food is not limited to general foodstuffs but also includes so-called health foods or medical foods such as nutritional supplements, foods with nutrient function claims, and foods for specified health uses. That is, the foods exemplified above may be provided as general foodstuffs or as health foods or medical foods.

The term "raw material of food" means a food material for production of foods. The raw material of food is not particularly limited as long as it can be used to produce foods. The raw material of food can be selected appropriately according to various conditions, such as the type of the food. Examples of the raw material of food include those typically used in the production of foods such as those exemplified above. Specific examples of the raw material of food include: cereals such as rice and wheat flour; seasoning ingredients such as sugars, inorganic salts, organic acids, nucleic acids, amino acids, and protein hydrolysates; dairy products such as milk, cheese, and butter; fruits; vegetables; meat; fish; eggs; spices; flavorings; oils and fats; alcohol; dietary fibers; and pH buffers.

The active ingredient may be added to the raw material of food at any stage of the food production process so long as the desired effect such as the flavor-improving effect is achieved. That is, the "raw material of food" to which the active ingredient is added may be one at any stage of the food production process. For example, the "raw material of food" to which the active ingredient is added may include finished food products prior to the addition of the active ingredient. The active ingredient may be added to the raw material of food either as is or after prepared into a desired form, such as a solution, as appropriate. The "addition of the active ingredient" may be broadly defined as any operation that brings the active ingredient into coexistence with the raw material of food. Components other than the active ingredient (e.g., cheese) may also be added to the raw material of food as appropriate. The descriptions regarding the addition of the active ingredient may be applied to the addition of components other than the active ingredient. The respective components (i.e., the active ingredient and optionally other components) may be added to the raw material of food all at once, or each may be added separately, or separately in any combination thereof. The order in which the respective components are added to the raw material of food is not particularly limited.

The addition amounts and addition ratios of the respective components (i.e., the active ingredient and optionally other components) in the method of the present invention are not particularly limited as long as the desired effect such as the flavor-improving effect can be obtained. The addition amounts and addition ratios of the respective components used in the method of the present invention can be appropriately set according to various conditions, such as the type of the raw material of food and the type of the food.

The active ingredient may be added to the raw material of food so that, for example, the concentration of the active ingredient at the time of eating falls within a desired range (e.g., such ranges of concentrations at the time of eating of the active ingredient as described below).

The concentration of the carboxylic acid at the time of eating may be, for example, 0.001 ppm (w/w) or higher, 0.01 ppm (w/w) or higher, 0.1 ppm (w/w) or higher, 0.2 ppm (w/w) or higher, 0.5 ppm (w/w) or higher, 1 ppm (w/w) or higher, 2 ppm (w/w) or higher, 5 ppm (w/w) or higher, 10 ppm (w/w) or higher, 20 ppm (w/w) or higher, 50 ppm (w/w) or higher, 100 ppm (w/w) or higher, 200 ppm (w/w) or higher, 500 ppm (w/w) or higher, 1,000 ppm (w/w) or higher, or 2,000 ppm (w/w) or higher, and may be 5,000 ppm (w/w) or lower, 2,000 ppm (w/w) or lower, 1,000 ppm (w/w) or lower, 500 ppm (w/w) or lower, 200 ppm (w/w) or lower, 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 0.5 ppm (w/w) or lower, or 0.2 ppm (w/w) or lower, or may be in a range defined by any non-contradictory combination of these minimum and maximum concentrations. The concentration of the carboxylic acid at the time of eating may specifically be, for example, 0.001 to 0.01 ppm (w/w), 0.1 to 0.2 ppm (w/w), 0.2 to 0.5 ppm (w/w), 0.5 to 1 ppm (w/w), 1 to 2 ppm (w/w), 2 to 5 ppm (w/w), 5 to 10 ppm (w/w), 10 to 20 ppm (w/w), 20 to 50 ppm (w/w), 50 to 100 ppm (w/w), 100 to 200 ppm (w/w), 200 to 500 ppm (w/w), 500 to 1,000 ppm (w/w), 1,000 to 2,000 ppm (w/w), or 2,000 to 5,000 ppm (w/w). The concentration of the carboxylic acid at the time of eating may specifically be, for example, 0.001 to 5,000 ppm (w/w), 0.01 to 4,000 ppm (w/w), 0.1 to 3,000 ppm (w/w), or 1.0 to 2,000 ppm (w/w). When the food of the present invention contains two or more types of carboxylic acids (e.g., when two or more types of carboxylic acids are added), the concentrations of those two or more types of carboxylic acids at the time of eating may be set, either independently or in total, within such ranges of concentration at the time of eating of the carboxylic acid as exemplified above. In one embodiment, for example, the concentration of 2-methylbutyric acid at the time of eating may be 0.001 to 5,000 ppm (w/w), 0.01 to 4,000 ppm (w/w), 0.1 to 3,000 ppm (w/w), or 1.0 to 2,000 ppm (w/w). In one embodiment, for example, the concentration of (S)-2-methylbutyric acid, in particular, at the time of eating may be 0.001 to 5,000 ppm (w/w), 0.01 to 4,000 ppm (w/w), 0.1 to 3,000 ppm (w/w), or 1.0 to 2,000 ppm (w/w). In one embodiment, for example, the concentrations of 3-methylbutyric acid and isobutyric acid at the time of eating may each independently be 0.1 to 1,000 ppm (w/w), 0.1 to 100 ppm (w/w), or 0.1 to 10 ppm (w/w). When the food of the present invention contains two or more types of carboxylic acids (e.g., when two or more types of carboxylic acids are added), the "concentration of carboxylic acid at the time of eating" shall mean the total concentration of those two or more types of carboxylic acids at the time of eating, unless especially specified.

When the food of the present invention contains two or more types of carboxylic acids, the contents of these two or more types of carboxylic acids in the food may or may not be the same. When the food of the present invention contains first and second carboxylic acids, the content of the second carboxylic acid in the food relative to 100 parts by weight of the first carboxylic acid contained in the food may be, for example, 0.1 parts by weight or more, 0.2 parts by weight or more, 0.5 parts by weight or more, 1 part by weight or more, 2 parts by weight or more, 5 parts by weight or more, 10 parts by weight or more, 20 parts by weight or more, 30 parts by weight or more, 50 parts by weight or more, or 70 parts by weight or more, and may be 100 parts by weight or less, 70 parts by weight or less, 50 parts by weight or less, 30 parts by weight or less, 20 parts by weight or less, 10 parts by weight or less, 5 parts by weight or less, 2 parts by weight or less, 1 part by weight or less, 0.5 parts by weight or less, or 0.2 parts by weight or less, or may be in a range defined by any non-contradictory combination of these minimum and maximum contents. The content of the second carboxylic acid in the food relative to 100 parts by weight of the first carboxylic acid contained in the food may specifically be, for example, 0.1 to 0.2 parts by weight, 0.2 to 0.5 parts by weight, 0.5 to 1 part by weight, 1 to 2 parts by weight, 2 to 5 parts by weight, 5 to 10 parts by weight, 10 to 20 parts by weight, 20 to 30 parts by weight, 30 to 50 parts by weight, 50 to 70 parts by weight, or 70 to 100 parts by weight. The content of the second carboxylic acid in the food relative to 100 parts by weight of the first carboxylic acid contained in the food may specifically be, for example, 0.1 to 100 parts by weight, 0.5 to 50 parts by weight, or 2 to 20 parts by weight. The first carboxylic acid may be any carboxylic acid, but may be, for example, 2-methylbutyric acid. The first carboxylic acid may specifically be, for example, (S)-2-methylbutyric acid, in particular. The second carboxylic acid may be any carboxylic acid other than the first carboxylic acid, but may be, for example, 3-methylbutyric acid or isobutyric acid. For example, when the food of the present invention contains 2-methylbutyric acid, 3-methylbutyric acid, and isobutyric acid, the contents of 3-methylbutyric acid and isobutyric acid in the food may be such amounts relative to 100 parts by weight of 2-methylbutyric acid contained in the food as exemplified above, either independently or in total. In one embodiment, when the active ingredient contained in the food of the present invention is produced by the production method of the present invention, the amount ratio (e.g., weight ratio or molar ratio) of 2-methylbutyric acid to 3-methylbutyric acid contained in the food of the present invention may be conveniently considered to be the same as the amount ratio (e.g., weight ratio or molar ratio) of isoleucine to leucine consumed in the culture of the production method of the present invention.

Further, the content of the carboxylic acid in the food of the present invention relative to 100 parts by weight of diacetyl contained in the food may be, for example, 0.5 parts by weight or more, 1 part by weight or more, 2 parts by weight or more, 3 parts by weight or more, 4 parts by weight or more, 5 parts by weight or more, 6 parts by weight or more, 8 parts by weight or more, 10 parts by weight or more, 12 parts by weight or more, 15 parts by weight or more, or 20 parts by weight or less, and may be 15 parts by weight or less, 12 parts by weight or less, 10 parts by weight or less, 8 parts by weight or less, 6 parts by weight or less, 5 parts by weight or less, 4 parts by weight or less, 3 parts by weight or less, 2 parts by weight or less, or 1 part by weight or less, or may be in a range defined by any non-contradictory combination of these minimum and maximum contents. The content of the carboxylic acid in the food of the present invention relative to 100 parts by weight of diacetyl contained in the food may specifically be, for example, 0.5 to 1 part by weight, 1 to 2 parts by weight, 2 to 3 parts by weight, 3 to 4 parts by weight, 4 to 5 parts by weight, 5 to 6 parts by weight, 6 to 8 parts by weight, 8 to 10 parts by weight, 10 to 12 parts by weight, 12 to 15 parts by weight, or 15 to 20 parts by weight. The content of the carboxylic acid in the food of the present invention relative to 100 parts by weight of diacetyl contained in the food may specifically be, for example, 0.5 to 20 parts by weight, 1 to 15 parts by weight, or 2 to 12 parts by weight. The content of the carboxylic acid in the food of the present invention relative to 100 parts by weight of diacetyl contained in the food may specifically be, for example, 0.5 to 8 parts by weight, 1 to 6 parts by weight, or 2 to 4 parts by weight.

Further, the content of the carboxylic acid in the food of the present invention relative to 100 parts by weight of acetoin contained in the food may be, for example, 10 parts by weight or more, 20 parts by weight or more, 50 parts by weight or more, 70 parts by weight or more, 100 parts by weight or more, 150 parts by weight or more, 200 parts by weight or more, 300 parts by weight or more, 400 parts by weight or more, 500 parts by weight or more, or 700 parts by weight or more, and may be 1,000 parts by weight or less, 700 parts by weight or less, 500 parts by weight or less, 400 parts by weight or less, 300 parts by weight or less, 200 parts by weight or less, 150 parts by weight or less, 100 parts by weight or less, 70 parts by weight or less, 50 parts by weight or less, or 20 parts by weight or less, or may be in a range defined by any non-contradictory combination of these minimum and maximum contents. The content of the carboxylic acid in the food of the present invention relative to 100 parts by weight of acetoin contained in the food may specifically be, for example, 10 to 20 parts by weight, 20 to 50 parts by weight, 50 to 70 parts by weight, 70 to 100 parts by weight, 100 to 150 parts by weight, 150 to 200 parts by weight, 200 to 300 parts by weight, 300 to 400 parts by weight, 400 to 500 parts by weight, 500 to 700 parts by weight, or 700 to 1,000 parts by weight. The content of the carboxylic acid in the food of the present invention relative to 100 parts by weight of acetoin contained in the food may specifically be, for example, 10 to 1,000 parts by weight, 20 to 700 parts by weight, or 50 to 500 parts by weight. The content of the carboxylic acid in the food of the present invention relative to 100 parts by weight of acetoin contained in the food may specifically be, for example, 10 to 300 parts by weight, 20 to 200 parts by weight, or 50 to 150 parts by weight.

The concentration of diacetyl at the time of eating may be, for example, 0.0001 ppm (w/w) or higher, 0.001 ppm (w/w) or higher, 0.01 ppm (w/w) or higher, 0.02 ppm (w/w) or higher, 0.05 ppm (w/w) or higher, 0.1 ppm (w/w) or higher, 0.2 ppm (w/w) or higher, 0.5 ppm (w/w) or higher, 1 ppm (w/w) or higher, 2 ppm (w/w) or higher, 5 ppm (w/w) or higher, 10 ppm (w/w) or higher, 20 ppm (w/w) or higher, or 50 ppm (w/w) or higher, and may be 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 0.5 ppm (w/w) or lower, 0.2 ppm (w/w) or lower, 0.1 ppm (w/w) or lower, 0.05 ppm (w/w) or lower, or 0.02 ppm (w/w) or lower, or may be in a range defined by any non-contradictory combination of these minimum and maximum concentrations. The concentration of diacetyl at the time of eating may specifically be, for example, 0.0001 to 0.001 ppm (w/w), 0.01 to 0.02 ppm (w/w), 0.02 to 0.05 ppm (w/w), 0.05 to 0.1 ppm (w/w), 0.1 to 0.2 ppm (w/w), 0.2 to 0.5 ppm (w/w), 0.5 to 1 ppm (w/w), 1 to 2 ppm (w/w), 2 to 5 ppm (w/w), 5 to 10 ppm (w/w), 10 to 20 ppm (w/w), 20 to 50 ppm (w/w), or 50 to 100 ppm (w/w). The diacetyl concentration at the time of eating may specifically be, for example, 0.0001 to 100 ppm (w/w), 0.001 to 80 ppm (w/w), 0.01 to 60 ppm (w/w), or 0.1 to 40 ppm (w/w).

The concentration of acetoin at the time of eating may be, for example, 0.001 ppm (w/w) or higher, 0.01 ppm (w/w) or higher, 0.1 ppm (w/w) or higher, 0.2 ppm (w/w) or higher, 0.5 ppm (w/w) or higher, 1 ppm (w/w) or higher, 2 ppm (w/w) or higher, 5 ppm (w/w) or higher, 10 ppm (w/w) or higher, 20 ppm (w/w) or higher, 50 ppm (w/w) or higher, 100 ppm (w/w) or higher, 200 ppm (w/w) or higher, 500 ppm (w/w) or higher, 1,000 ppm (w/w) or higher, or 2,000 ppm (w/w) or higher, and may be 5,000 ppm (w/w) or lower, 2,000 ppm (w/w) or lower, 1,000 ppm (w/w) or lower, 500 ppm (w/w) or lower, 200 ppm (w/w) or lower, 100 ppm (w/w) or lower, 50 ppm (w/w) or lower, 20 ppm (w/w) or lower, 10 ppm (w/w) or lower, 5 ppm (w/w) or lower, 2 ppm (w/w) or lower, 1 ppm (w/w) or lower, 0.5 ppm (w/w) or lower, or 0.2 ppm (w/w) or lower, or may be in a range defined by any non-contradictory combination of these minimum and maximum concentrations. The concentration of acetoin at the time of eating may specifically be, for example, 0.001 to 0.01 ppm (w/w), 0.1 to 0.2 ppm (w/w), 0.2 to 0.5 ppm (w/w), 0.5 to 1 ppm (w/w), 1 to 2 ppm (w/w), 2 to 5 ppm (w/w), 5 to 10 ppm (w/w), 10 to 20 ppm (w/w), 20 to 50 ppm (w/w), 50 to 100 ppm (w/w), 100 to 200 ppm (w/w), 200 to 500 ppm (w/w), 500 to 1,000 ppm (w/w), 1,000 to 2,000 ppm (w/w), or 2,000 to 5,000 ppm (w/w). The concentration of acetoin at the time of eating may specifically be, for example, 0.001 to 5,000 ppm (w/w), 0.01 to 4,000 ppm (w/w), 0.1 to 3,000 ppm (w/w), or 1.0 to 2,000 ppm (w/w).

The descriptions regarding the addition of the active ingredients are also applicable to the addition of the composition of the present invention. For example, the composition of the present invention may be added so that any of the addition amounts of the active ingredient exemplified above is obtained.

In one embodiment, the food of the present invention may not contain cheese. That is, the food of the present invention may be produced so as not to contain cheese.

In one embodiment, the food of the present invention may contain cheese. That is, the food of the present invention may be produced to contain cheese. The food containing cheese may be produced by, for example, adding cheese. That is, the method of the present invention may further comprise adding cheese to a raw material of food. The addition of cheese may be performed similarly to the addition of the active ingredient. Further, the food containing cheese may be produced by, for example, using a raw material of food containing cheese. That is, the raw material of food may contain cheese.

The food of the present invention is typically a food containing cheese, and may be one with a reduced cheese content. The "food with a reduced cheese content" means a food with a cheese content lower than usual (i.e., food with a cheese content lower than the usual cheese content), and includes foods containing no cheese at all. A food containing a normal amount of cheese (i.e., food with a normal cheese content) is also referred to as "normal cheese-containing food". That is, the "food with a reduced cheese content" may specifically mean a food of the same type as a normal cheese-containing food, but with a cheese content lower than that of the normal cheese-containing food. More specifically, the "food with a reduced cheese content" may mean a food of the same type as a normal cheese-containing food where the cheese content is 0.9 times or less, 0.8 times or less, 0.7 times or less, 0.6 times or less, 0.5 times or less, 0.4 times or less, 0.3 times or less, 0.2 times or less, or 0.1 times or less of that of the normal cheese-containing food, and may also include foods containing no cheese at all. Examples of such a food with a reduced cheese content include such foods as listed above typically containing cheese, but produced to have a lower cheese content than usual or to contain no cheese. Examples of normal cheese-containing foods include cheese-containing foods such as those exemplified above, which have been produced to contain a normal amount of cheese. According to the present invention, for example, reduction in cheese flavor due to reduced cheese content may be compensated for. That is, "impartation of cheese flavor" may also include compensating for reduction in cheese flavor caused by reduced cheese content.

The method of the present invention may comprise the step of producing the active ingredient prior to the step of utilizing the active ingredient (e.g., a step of adding the active ingredient to a raw material of food). The active ingredient utilized in the method of the present invention is produced by the production method of the present invention. Therefore, the step of producing the active ingredient in the method of the present invention may be the step of producing the active ingredient by the production method of the present invention. Specifically, the step of producing the active ingredient in the method of the present invention may be the culture step of the production method of the present invention. The step of producing the active ingredient in the method of the present invention may specifically be a step of obtaining a culture containing carboxylic acid, diacetyl, and acetoin by culturing an active ingredient-producing bacterium having a carboxylic acid-producing ability, a diacetyl-producing ability, and an acetoin-producing ability in a medium containing a precursor. The active ingredient utilized in the method of the present invention may be produced as, for example, the composition of the present invention. Therefore, the step of producing the active ingredient in the method of the present invention may be, for example, the step of producing the composition of the present invention by the production method of the present invention.

### <4> Use of active ingredient

The present invention also discloses use of the active ingredient in the applications exemplified above. That is, the present invention discloses, for example, use of the active ingredient for improving flavor of foods or for producing foods, and use of the active ingredient in the production of compositions for improving flavor of foods or for producing foods.

The present invention also discloses the active ingredient for use in the applications exemplified above. That is, the present invention discloses, for example, the active ingredient for use in improving flavor of foods or for producing foods, and the active ingredient for use in production of compositions for improving flavor of foods or for producing foods.

### Examples

Hereafter, the present invention will be explained more specifically with reference to non-limiting examples. All the precursors (amino acids) used in the examples are L-amino acids.

### Example 1: Fermentation test using Bacillus bacteria (evaluation of difference of strain)

Two strains listed in Table 1 were used as the *Bacillus* bacterium. These were each inoculated from a glycerol stock into a liquid medium (yeast extract 1%, peptone 1%, Glc 1%) and cultured with shaking at 30°C and 120 rpm for 20 hours to prepare a pre-culture broth. Then, a main fermentation medium 1 was prepared by using the composition shown in Table 2. Calcium carbonate for pH buffering (1 g) and the main fermentation medium 1 (10 mL) were added to one 500-mL Sakaguchi flask, the pre-culture broth (1 mL) was added thereto, and main fermentation was conducted by shaking culture at 30°C and 120 rpm for 48.5 hours.

The results are shown in Table 3. Both *Bacillus subtilis and Bacillus amyloliquefaciens* consumed the sugar (glucose) and Ile, and simultaneously produced 2-methylbutyric acid (2MB), acetoin, and diacetyl. The balance of the produced components differed between the two strains. Note that 2MB and 3-methylbutyric acid (IVA) were difficult to separate, and therefore the quantified 2MB might contain IVA. However, since Ile is a precursor of 2MB, the major part of the quantified 2MB was considered to be 2MB. From the above, it was revealed that the components of the active ingredient can be collectively produced by using a *Bacillus* bacterium.

**[Table 1]**

| Table 1: List of strains | | |
|---|---|---|
| | Strain | Deposition No. |
| 1 | *Bacillus subtilis* | NBRC 13719 |
| 2 | *Bacillus amyloliquefaciens* | NBRC 15535 |

**[Table 2]**

| Table 2: Medium composition | | |
|---|---|---|
| Group A | Medium 1 | |
| MgSO₄ | 1.0 | g/L |
| Glucose | 60.0 | g/L |
| Group B | | |
| Yeast Extract | 15.0 | g/L |
| Peptone | 20.0 | g/L |
| KH₂PO₄ | 4.0 | g/L |
| Ile | 15.0 | g/L |

| | | |
|---|---|---|
| *The medium components of the groups A and B were separately sterilized (120°C, 20 minutes) and then mixed to prepare the media. | | |

**[Table 3]**

| Table 3: Results of fermentation (fermentation for 48 hours) | | | | | | | | |
|---|---|---|---|---|---|---|---|---|
| No . | Strain | Medium | Added sugar | Added amino acid | 2MB* (ppm) | Acetoin (ppm) | Diacetyl (ppm) | Isobutyric acid (ppm) |
| 1 | *Bacillus subtilis* | Medium 1 | Glc | Ile | 7999.9 | 9456.6 | 255.1 | 330.5 |
| 2 | *Bacillus amylolique faciens* | Medium 1 | Glc | Ile | 3335.6 | 12745.9 | 360.5 | 274.3 |

| | | | | | | | | |
|---|---|---|---|---|---|---|---|---|
| *Since 2MB and 3-methylbutyric acid (IVA) were difficult to separate, and therefore the quantified 2MB might also contain IVA. | | | | | | | | |

### Example 2: Fermentation test using Bacillus bacterium (evaluation of difference in medium composition)

(1) *Bacillus subtilis* mentioned in Table 1 was used as the *Bacillus* bacterium. It was inoculated from a glycerol stock into a liquid medium (yeast extract 1%, peptone 1%, Glc 1%) and cultured with shaking at 30°C and 120 rpm for 20 hours to prepare a pre-culture broth. Then, main fermentation medium 1, main fermentation medium 2, and main fermentation medium 3 were prepared by using the compositions mentioned in Table 4. For the media 1 and 3 supplemented with sugar, 1 g of calcium carbonate for pH buffering and 10 mL of each main fermentation medium were added to a single 500-mL Sakaguchi flask, 1 mL of the pre-culture broth was added thereto, and main fermentation was conducted with shaking at 30°C and 120 rpm for 48.5 hours. For the medium 2 not supplemented with sugar, 10 mL of the main fermentation medium was added to a single 500-mL Sakaguchi flask without adding calcium carbonate, 1 mL of the pre-culture broth was added thereto, and main fermentation was conducted with shaking at 30°C and 120 rpm for 48.5 hours.

The results are shown in Table 5. The data for No. 1 in Table 5 are reproduction of the data for No. 1 in Table 3. The initial concentrations of the respective amino acids include those originated in the yeast extract and peptone. Acetoin and diacetyl were produced in the medium 1 and medium 3 supplemented with sugar, but were not produced in the medium 2 not supplemented with sugar. In the medium 3 supplemented with Ile, Leu, and Val, increased production amounts of 2-methylbutyric acid (2MB) and isobutyric acid were observed compared with the medium 1 and medium 2 not supplemented with Leu and Val. Note that 2MB and 3-methylbutyric acid (IVA) were difficult to separate, and therefore the quantified 2MB might also contain IVA. However, since Ile is a precursor of 2MB, it is considered that the major part of the quantified 2MB was 2MB. Further, since Leu is a precursor of IVA, it is considered that the addition of Leu also contributed to the increased IVA production. From the above, it was revealed that addition of sugar is effective for the production of acetoin and diacetyl. It was also revealed that addition of carboxylic acid precursor is effective for the production of carboxylic acid.

**[Table 4]**

| Table 4: Medium composition | | | | | |
|---|---|---|---|---|---|
| Group A | Medium 1 | Medium 2 | Medium 3 | Medium 4 | |
| MgSO₄ | 1.0 | 1.0 | 1.0 | 1.0 | g/L |
| Glucose | 60.0 | | 60.0 | 60.0 | g/L |
| Group B | | | | | |
| Yeast Extract | 15.0 | 15.0 | 15.0 | 15.0 | g/L |
| Peptone | 20.0 | 20.0 | 20.0 | 20.0 | g/L |
| KH₂PO₄ | 4.0 | 4.0 | 4.0 | 4.0 | g/L |
| Ile | 15.0 | 15.0 | 15.0 | | g/L |
| Leu | | | 1.5 | 15.0 | g/L |
| Val | | | 1.5 | | g/L |

| | | | | | |
|---|---|---|---|---|---|
| *The medium components of the groups A and B were separately sterilized (120°C, 20 minutes) and then mixed to prepare the media. | | | | | |

**[Table 5]**

| Table 5: Results of fermentation using *Bacillus subtilis* (fermentation for 48 hours) | | | | | | | | |
|---|---|---|---|---|---|---|---|---|
| No. | Strain | Medium | Added sugar | Added amino acid | 2MB* (ppm) | Acetoin (ppm) | Diacetyl (ppm) | Isobutyric acid (ppm) |
| 1 | *Bacillus subtilis* | Medium 1 | Glc | Ile | 7999.9 | 9456.6 | 255.1 | 330.5 |
| 2 | *Bacillus subtilis* | Medium 2 | - | Ile | 6760.0 | Not detected | Not detected | 312.7 |
| 3 | *Bacillus subtilis* | Medium 3 | Glc | Ile + Leu + Val | 8834.7 | 9831.9 | 316.6 | 359.7 |

| No. | Consumed Ile (g/L) | Consumed Leu (g/L) | Consumed Val (g/L) | Initial Ile (g/L) | Initial Leu (g/L) | Initial Val (g/L) |
|---|---|---|---|---|---|---|
| 1 | 8.2 | 1.1 | 0.0 | 14.4 | 1.4 | 1.0 |
| 2 | 7.9 | 1.1 | 0.1 | 15.1 | 1.4 | 1.1 |
| 3 | 9.1 | 1.8 | 0.5 | 15.6 | 3.0 | 2.5 |

| | | | | | | |
|---|---|---|---|---|---|---|
| *Since 2MB and 3-methylbutyric acid (IVA) were difficult to separate, and therefore the quantified 2MB might also contain IVA. | | | | | | |

(2) Although isobutyric acid can be generated from Val, the consumption of Val was low in the experiment of (1). Therefore, the possibility of generation of isobutyric acid from Ile or Leu was evaluated.

Culture was performed in the same manner as in the experiment of (1) by using the main fermentation medium 1 and main fermentation medium 4 shown in Table 4. The consumed amount of each amino acid after 48 hours of the fermentation was quantified. The production amounts of isobutyric acid after 24 and 48 hours of the fermentation were quantified as GC/MS peak areas.

The results are shown in Table 6. In both media, amino acids (in particular, Ile or Leu added) were consumed, and isobutyric acid production was observed. Notably, both the total amino acid consumption and isobutyric acid production were higher in the main medium 1 (Ile-added medium). From these results, it is considered possible that isobutyric acid is produced from Ile and/or Leu.

**[Table 6]**

| Table 6: Results of fermentation using *Bacillus subtilis* | | | | | |
|---|---|---|---|---|---|
| No. | Consumed Val (g/L) | Consumed Ile (g/L) | Consumed Leu (g/L) | Isobutyric acid peak area | Isobutyric acid peak area |
| | | | | (fermentation for 24 hours) | (fermentation for 48 hours) |
| 1 (Ile-added medium) | 0.34 | 10.21 | 0.88 | 3.01E+08 | 4.16E+08 |
| 2 (Leu-added medium) | 0.3 | 0.58 | 7.13 | 2.80E+08 | 3.58E+08 |

### Example 3: Sensory evaluation of fermentation broth (evaluation of difference in strain)

A sensory evaluation was conducted on the two types of fermentation broths prepared in Example 1. The evaluation system used a commercial product containing cheese, "Knorr (registered trademark) Cup Soup, 4 Types of Cheese Thick Potage (Ajinomoto)", and a simulated sauce without cheese flavor that simulated plant-based cheese. The simulated sauce was prepared by using the composition shown in Table 7. The respective raw materials were placed in a mixer, blended for approximately 1 minute, and then heated at 60°C for 3 minutes to prepare the simulated sauce. The fermentation broths were added to the potage and simulated sauce at a concentration of 0.3%, and evaluation was conducted on the basis of consensus among five experts. Cheddar Cheese Powder (Kerry, CR-15270) was used as the positive control (PC).

The results are shown in Tables 8 and 9. Addition of either one of the fermentation broths of *B. subtilis* and *B. amyloliquefaciens* enhanced the cheese flavor. In the evaluation using potage, which inherently had cheese flavor, the fermentation broth of *B. amyloliquefaciens* provided strong yogurt flavor and poor balance, but the *B. subtilis* fermentation broth enhanced the cheese flavor in a well-balanced manner. In the evaluation using the simulated sauce without cheese flavor, both fermentation broths imparted well-balanced cheese flavor. From the above, it was revealed that fermentation broths from *Bacillus* bacteria are effective for imparting cheese flavor.

**[Table 7]**

| Table 7: Composition of simulated sauce | |
|---|---|
| Raw material | Formulation amount (%) |
| Potato starch | 13.0 |
| Shiratama flour (glutinous | 5.0 |
| rice flour) | |
| Almond Protein | 8.0 |
| Salt | 0.3 |
| Coconut Oil | 24.3 |
| Water | 49.5 |
| Total | 100 |

**[Table 8]**

| Table 8: Results of sensory evaluation (evaluation using potage) | | | | |
|---|---|---|---|---|
| | Test group 1 | Test group 2 | Test group 3 | Test group 4 |
| Sample used | No addition | Cheddar cheese powder | Fermentation broth of *B. subtilis* | Fermentation broth of *B. amyloliquefaciens* |
| Addition ratio | - | 1.00% | 0.30% | 0.30% |
| Cheese flavor | 1.0 | 5.0 | 4.5 | 3.5 |
| Note | - | Richness and cheesiness were improved. | Richness and cheesiness were improved. Balance was good, and cheese flavor was enhanced. | Yogurt flavor was stronger than the others. Balance was bad despite high titer. |

| | | | | |
|---|---|---|---|---|
| *The scores of cheese flavor were determined for the respective test groups on the basis of the scores of cheese flavors of the test group 1 where any fermentation broth was not added and the test group 2 where cheddar cheese powder (PC) was added, which were taken as "1" and "5", respectively. | | | | |

**[Table 9]**

| Table 9: Results of sensory evaluation (evaluation using simulated sauce) | | | | |
|---|---|---|---|---|
| | Test group 1 | Test group 2 | Test group 3 | Test group 4 |
| Sample used | No addition | Cheddar cheese powder | Fermentation broth of *B. subtilis* | Fermentation broth of *B. amyloliquefaciens* |
| Addition ratio | - | 1.00% | 0.30% | 0.30% |
| Cheese flavor | 1.0 | 5.0 | 4.5 | 4.0 |
| Note | No cheese flavor | Richness and cheesiness were imparted. | Richness and cheesiness were imparted. Initial flavor was slightly weak, but close to that of test group 2. Sauce was creamy, but bitterness was felt. | Cheesiness was imparted. Yogurt flavor was strong, but the balance was not bad compared with the system of potage. |

| | | | | |
|---|---|---|---|---|
| *The scores were determined for the respective test groups on the basis of the scores of cheese flavors of the test group 1 where any fermentation broth was not added and the test group 2 where cheddar cheese powder (PC) was added, which were taken as "1" and "5", respectively. | | | | |

### Example 4: Sensory evaluation of fermentation broth (evaluation of difference in medium composition)

A sensory evaluation was conducted on the three types of fermentation broths prepared in Example 2. The evaluation system used a simulated sauce simulating plant-based cheese without cheese flavor. The simulated sauce was prepared by using the composition shown in Table 7. The respective raw materials were placed in a mixer, blended for approximately 1 minute, and then heated at 60°C for 3 minutes to prepare the simulated sauce. The fermentation broths were each added to the simulated sauce at a concentration of 0.3%, and the samples were evaluated on the basis of consensus of five experts. Cheddar cheese powder (Kerry, CR-15270) was used as the positive control (PC).

The results are shown in Table 10. All the fermentation broths imparted cheese flavor when they were added, but the fermentation broth obtained with the medium 2 (fermentation broth not containing diacetyl and acetoin) showed a weaker cheese flavor-imparting effect compared with the fermentation broths obtained with the medium 1 and medium 3 (fermentation broths containing diacetyl and acetoin). The fermentation broths obtained with the medium 1 and medium 3 (fermentation broths containing diacetyl and acetoin) both imparted a well-balanced cheese flavor, with the effect being particularly pronounced with the fermentation broth obtained with the medium 3. From the above, it was clarified that addition of a combination of carboxylic acid, diacetyl, and acetoin is more effective for imparting cheese flavor than addition of carboxylic acid alone. It was also clarified that fermentation broths of *Bacillus* bacteria produced with media supplemented with sugar, Ile, Leu, and Val are particularly effective for imparting cheese flavor.

### [Table 10]

**Table 10**

| | Test group 1 | Test group 2 | Test group 3 | Test group 4 | Test group 5 |
|---|---|---|---|---|---|
| Sample used | No addition | Cheddar cheese powder | Fermentation broth obtained with medium 2 | Fermentation broth obtained with medium 1 | Fermentation broth obtained with medium 3 |
| Addition ratio | - | 1.00% | 0.30% | 0.30% | 0.30% |
| Cheese flavor | 1.0 | 5.0 | 3.5 | 4.5 | 5.0 |
| Note | No cheese flavor | Richness and cheesiness were imparted. | Cheese-like vibrant sweet flavor was imparted. Type of flavor was similar to that of cheese, but the effect was slightly weaker. | Richness and cheesiness were imparted. Initial flavor was slightly weak, but closer to that of test group 2. | More complex flavor compared with test group 4. Strongest cheese flavor was imparted. |

| | | | | | |
|---|---|---|---|---|---|
| *The scores were determined for the respective test groups on the basis of the scores of cheese flavors of the test group 1 where any fermentation broth was not added and the test group 2 where cheddar cheese powder (PC) was added, which were taken as "1" and "5", respectively. | | | | | |

## Claims

1. A method for producing a carboxylic acid, diacetyl, and acetoin, comprising
the step of culturing a *Bacillus* bacterium having a carboxylic acid-producing ability, a diacetyl-producing ability, and an acetoin-producing ability in a culture medium containing a precursor of the carboxylic acid and a sugar to obtain a culture containing the carboxylic acid, diacetyl, and acetoin, wherein
the carboxylic acid is selected from the group consisting of 2-methylbutyric acid, 3-methylbutyric acid, isobutyric acid, and a combination thereof,
the precursor of 2-methylbutyric acid is isoleucine, the precursor of 3-methylbutyric acid is leucine, and the precursor of isobutyric acid is selected from the group consisting of isoleucine, leucine, valine, and a combination thereof.

2. The method according to claim 1, wherein 2-methylbutyric acid is (S)-2-methylbutyric acid and/or (R)-2-methylbutyric acid, and
the precursor of (S)-2-methylbutyric acid and the precursor of (R)-2-methylbutyric acid are L-isoleucine and D-isoleucine, respectively.

3. The method according to claim 1, wherein the carboxylic acid, diacetyl, and acetoin are produced as a composition comprising the carboxylic acid, diacetyl, and acetoin.

4. The method according to claim 3, wherein the composition comprises the culture or a processed product thereof.

5. The method according to claim 3, wherein the composition comprises a dried product of the culture or a dried product of the supernatant of the culture.

6. The method according to claim 3, wherein content of the carboxylic acid in the composition is 10 ppm (w/w) or higher.

7. The method according to claim 3, wherein content of diacetyl in the composition is 0.5 ppm (w/w) or higher.

8. The method according to claim 3, wherein content of acetoin in the composition is 10 ppm (w/w) or higher.

9. The method according to any one of claims 1 to 8, wherein the bacterium is *Bacillus subtilis*, *Bacillus amyloliquefaciens*, *Bacillus pumilus*, *Bacillus licheniformis*, *Bacillus megaterium*, *Bacillus brevis*, *Bacillus polymixa*, *Bacillus stearothermophilus*, or *Bacillus velezensis*.

10. The method according to any one of claims 1 to 8, wherein the bacterium is *Bacillus subtilis* or *Bacillus amyloliquefaciens*.

11. The method according to any one of claims 1 to 8, wherein isoleucine content in the medium is 0.1 to 5% (w/w).

12. The method according to any one of claims 1 to 8, wherein leucine content in the medium is 0.1 to 5% (w/w).

13. The method according to any one of claims 1 to 8, wherein valine content in the medium is 0.1 to 5% (w/w).

14. The method according to any one of claims 1 to 8, wherein content of the sugar in the medium is 1 to 50% (w/w).

15. The method according to any one of claims 1 to 8, wherein the sugar is glucose.

16. The method according to claim 3, wherein the composition is a composition for improving flavor of a food.

17. The method according to claim 16, wherein the improvement of flavor is imparting cheese flavor.

18. The method according to claim 3, wherein the composition is a seasoning.

19. The method according to any one of claims 1 to 8, wherein at least 2-methylbutyric acid is produced.

20. The method according to claim 19, wherein 3-methylbutyric acid is further produced.

21. The method according to claim 19, wherein isobutyric acid is further produced.

22. A composition produced by the method according to claim 3.

23. A composition for improving flavor of a food, comprising
the following components (A), (B), and (C):
(A) a carboxylic acid selected from the group consisting of 2-methylbutyric acid, 3-methylbutyric acid, isobutyric acid, and a combination thereof;
(B) diacetyl; and
(C) acetoin.

24. The composition according to claim 23, wherein the components (A), (B), and (C) have been produced by the method according to any one of claims 1 to 8.

25. The composition according to claim 23, which comprises at least 2-methylbutyric acid.

26. The composition according to claim 25, which further comprises 3-methylbutyric acid.

27. The composition according to claim 25, which further comprises isobutyric acid.

28. The composition according to claim 23, wherein the improvement of flavor is imparting cheese flavor.

29. A method for improving flavor of a food, comprising:
the step of adding the following components (A), (B), and (C) to a raw material of the food:
(A) a carboxylic acid selected from the group consisting of 2-methylbutyric acid, 3-methylbutyric acid, isobutyric acid, and a combination thereof;
(B) diacetyl; and
(C) acetoin.

30. The method according to claim 29, wherein the components (A), (B), and (C) have been produced by the method according to any one of claims 1 to 8.

31. The method according to claim 29, which comprises the step of producing the components (A), (B), and (C) by the method according to any one of claims 1 to 8 prior to the step mentioned above.

32. The method according to claim 29, wherein the component (A) is added so that the concentration thereof at the time of eating is 0.001 to 5,000 ppm (w/w).

33. The method according to claim 29, wherein the component (B) is added so that the concentration thereof at the time of eating is 0.0001 to 100 ppm (w/w).

34. The method according to claim 29, wherein the component (C) is added so that the concentration thereof at the time of eating is 0.001 to 5,000 ppm (w/w).

35. The method according to claim 29, wherein at least 2-methylbutyric acid is added.

36. The method according to claim 35, wherein 3-methylbutyric acid is further added.

37. The method according to claim 35, wherein isobutyric acid is further added.

38. The method according to claim 29, wherein the improvement of flavor is imparting cheese flavor.
